(19) Europäisches Patentamt
European Patent Office
Office européen des brevets

(11) Publication number: **0 208 855 B1**

# (12) EUROPEAN PATENT SPECIFICATION

(45) Date of publication of patent specification: **06.03.91**

(51) Int. Cl.$^5$: **C07D 401/04**, C07D 221/16, C07D 213/81, A61K 31/435

(21) Application number: **86106257.8**

(22) Date of filing: **07.05.86**

**(54) Process for preparing piperidylidene dihydrodibenzo(a,d)cycloheptenes and aza derivatives thereof, compounds obtained by such process and the use of such compounds for preparing useful pharmaceutical compositions.**

(30) Priority: **13.05.85 US 733428**
**12.03.86 US 838974**
**12.03.86 US 839016**

(43) Date of publication of application:
**21.01.87 Bulletin 87/04**

(45) Publication of the grant of the patent:
**06.03.91 Bulletin 91/10**

(84) Designated Contracting States:
**AT BE CH DE FR GB IT LI LU NL SE**

(56) References cited:
**EP-A- 0 152 897**
**FR-A- 2 247 235**
**US-A- 3 717 647**
**CH-A- 535 769**
**GB-A- 1 065 191**
**US-A- 4 282 233**

(73) Proprietor: **SCHERING CORPORATION**
**2000 Galloping Hill Road**
**Kenilworth New Jersey 07033(US)**

(72) Inventor: **Schumacher, Doris Pickel**
**76 Edgewood Drive**
**Florham Park New Jersey 07932(US)**
Inventor: **Villani, Frank J.**
**22 Oakland Terrace**
**Fairfield New Jersey 07083(US)**
Inventor: **Wong, Jesse Kwok-Keung**
**547 Andress Terrace**
**Union New Jersey 07083(US)**
Inventor: **Murphy, Bruce Lee**
**969 Bloomfield Avenue**
**Glen Ridge New Jersey 07028(US)**
Inventor: **Clark, Jon Edward**
**501 Gove Avenue**
**Highland Park New Jersey 08904(US)**

(74) Representative: **von Kreisler, Alek, Dipl.-Chem. et al**
**Deichmannhaus am Hauptbahnhof**
**W-5000 Köln 1(DE)**

**EP 0 208 855 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an improved process in the preparation of compounds of the general formula I

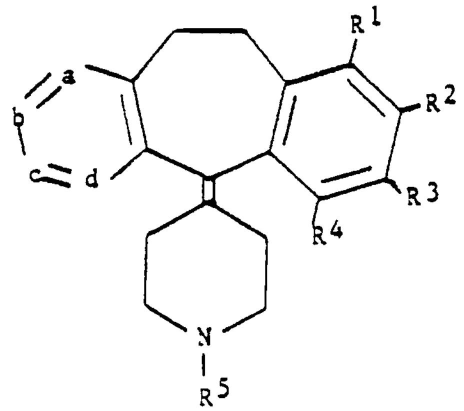

I

wherein a, b, c, and d represent CH or one of a, b, c and d represents N and the others represent CH;

$R^1$, $R^2{}_1$ $R^3$ and $R^4$ may be the same or different and each independently represents hydrogen, alkyl having from 1 to 6 carbon atoms, fluoro, chloro, bromo iodo, nitro, alkoxy having from 1 to 6 carbon atoms or trifluoromethyl;

$R^5$ is methyl, -H or $-COOR^7$, wherein $R^7$ represents $C_1$ to $C_{12}$ alkyl, substituted $C_1$ to $C_{12}$ alkyl, $C_2$ to $C_{12}$ alkenyl, substituted $C_2$ to $C_{12}$ alkenyl, phenyl, substituted phenyl, $C_7$ to $C_{10}$ phenylalkyl or $C_7$ to $C_{10}$ phenylalkyl wherein the phenyl moiety is substituted, or $R^7$ is 2-, 3-, or 4-piperidyl or N-substituted piperidyl, wherein the substituents on said substituted $C_1$ to $C_{12}$ alkyl and on said substituted $C_2$ to $C_{12}$ alkenyl are selected from amino or substituted amino and the substituents on said substituted amino are selected from $C_1$ to $C_6$ alkyl, the substituents on said substituted phenyl and on said substituted phenyl moiety of the $C_7$ to $C_{10}$ phenylalkyl are selected from $C_1$ to $C_4$ alkyl and halo, and the substituent on said N-substituted piperidyl is $C_1$ to $C_4$ alkyl;

and pharmaceutically acceptable salts of such compounds, whereby a compound of the general formula II

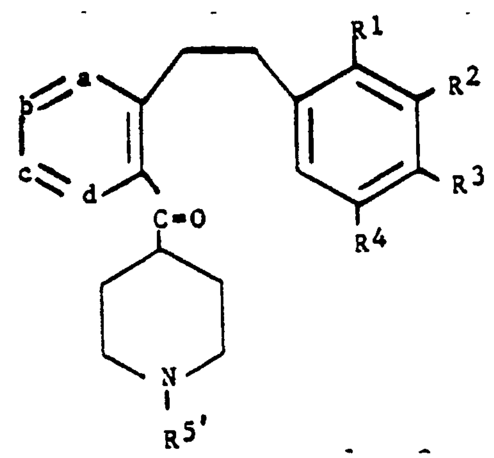

II

wherein a, b, c, d, $R^1$, $R^2$, $R^3$ and $R^4$ are as defined above and

$R^{5'}$ is as defined for $R^5$ above or represents are N-protecting group, is subjected to an intramolecular condensation followed, if necessary or if desired, by one or more of the following steps

a) removal of any N-protecting group represented by $R^{5'}$;

b) transformation of a compound wherein $R^5$ is methyl or $-COOR^7$ into a compound wherein $R^5$ is hydrogen;

c) transformation of a compound wherein $R^5$ is hydrogen into a compound wherein $R^5$ is $-COOR^7$or methyl;

d) transformation of a compound wherein $R^5$ is methyl into a compound wherein $R^5$ is $-COOR^7$; or

e) transformation of a compound of formula I obtained according to any one of the steps above into a

pharmaceutically acceptable salt;

characterized in that the intramolecular condensation is performed by treating the compound of formula II with a super acid having a Hammett acidity function of minus 12 or lower.

The starting compounds of formula II may be prepared by the following procedures:

A compound of formula III

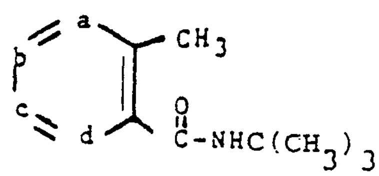

III

is reacted A: with a compound of formula IV

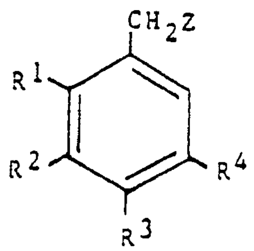

IV

in the presence of base to produce a compound of formula V

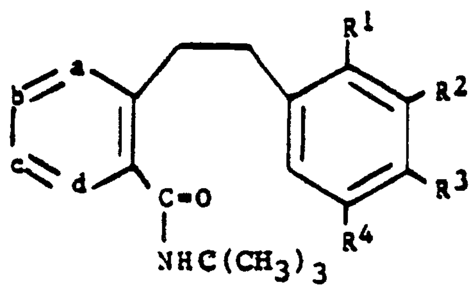

V;

reacting the compound of formula V above with a dehydrating agent to produce a compound of formula VI

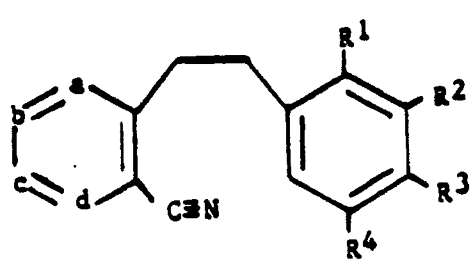

VI;

reacting the compound of formula VI with a compound of formula VII

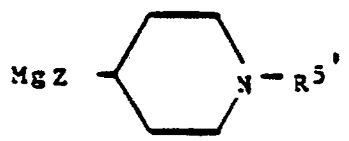

VII

and hydrolyzing the product thereof.

In the above reaction scheme a, b, c, d and $R^1$ to $R^{5'}$ are as defined above and Z represents Cl, Br or I.

The compounds of formula V are novel compounds. Except for being useful for making the starting compounds of formula II for the process claimed herein, they can also easily and with high yields be cyclized to compounds of formula IX which are the starting compounds for the conventional method of making compounds of formula I (illustrated for preparation of compounds of formula XII defined hereinafter):

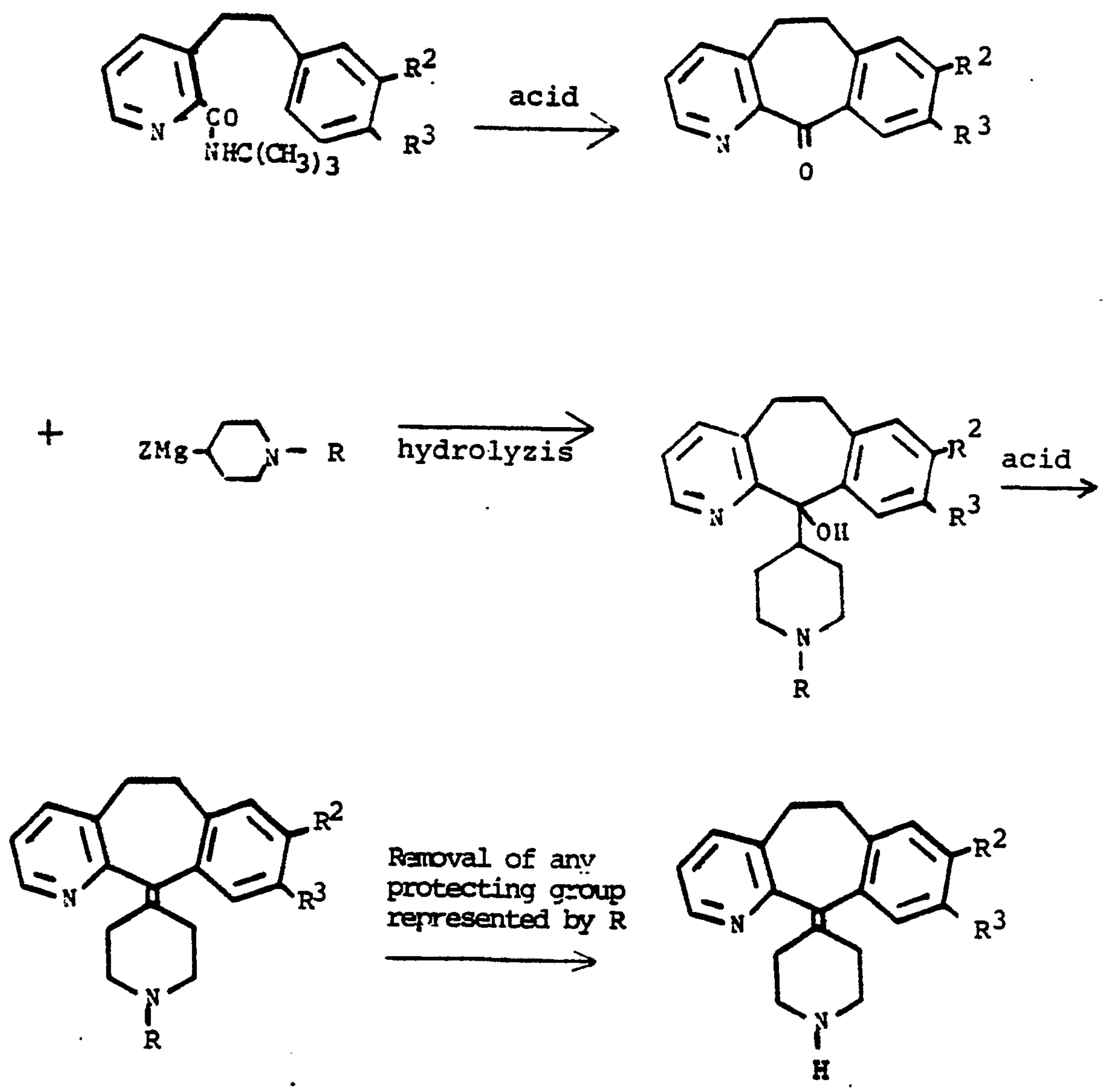


XII

The ring closure (V → IX) may also here advantageously be performed by means of a super acid, but other strong acids are also applicable.

Processes for making compounds of formula I have been disclosed in the art, e.g. U.S. Patent Nos.

3.326.924 and 3.717.647 and Villani et al., Journal of Medical Chemistry, 1972, Vol. 15, No. 7, pp 750-754. The possibility of making an intramolecular condensation directly from compounds II to compounds I has also been suggested (e.g. Swiss Patent No. 535,769). The reagent proposed in said patent is polyphosphoric acid. This procedure gives very poor yields and the reaction results in a mixture of compounds from which the desired product can hardly be isolated. The process claimed herein provides much higher yields with a low amount of impurities being formed.

The compounds of formula III are either known or may be obtained, for example, by a Ritter reaction in which a tertiary butyl compound is reacted with a cyanotoluene or cyano-methyl-pyridine compound, e.g.:

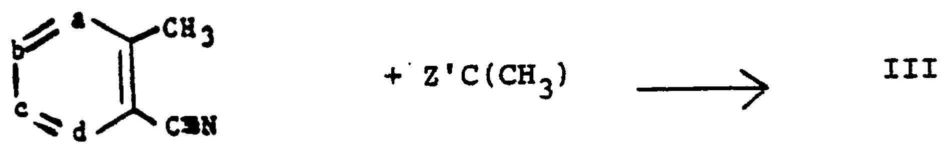


(Z' represents OH, Cl, Br etc.)

This reaction is generally performed in an acid such as concentrated sulfuric acid or concentrated sulfuric acid in glacial acetic acid. Suitable tertiary butyl compounds include, but are not limited to, t-butyl alcohol, t-butyl chloride, t-butyl bromide, t-butyl iodide, isobutylene or any other compounds which under hydrolytic conditions forms t-butyl carboxamides with cyano compounds. The temperature of the reaction will vary depending on the reactants, but generally is conducted in the range of from about 50° to about 100°C with t-butyl alcohol. The reaction may be performed with inert solvents but is usually run neat.

The compound formula III is reacted with an appropriate benzyl halide of formula IV in the presence of a base to form the compound of formula V above. The selection of the benzyl halides depends on the substitutes $R^1$ to $R^4$ which are desired and include, but are not limited to, benzyl chloride, 3-chloro-benzyl chloride, 3-fluoro-benzyl chloride, 3,4-dichlorobenzylchloride, 4-fluoro-benzyl chloride, 3-nitro-benzyl chloride, 3-methyl-benzyl chloride, etc. Any suitable base can be employed, e.g., an alkyl lithium compound such as n-butyl lithium in tetrahydrofuran (THF). Preferably the base has a $pK_a$ of greater than 20 and more preferably greater than 30. This reaction can be conducted at any suitable temperature, e.g., temperatures of from about -78°C to about 30°C, preferably from about -40°C to about -30°C. The reaction can be performed in any suitable inert solvent such as THF, diethyl ether, etc.

The amide of formula V is converted to the cyano compound of formula VI by the use of a suitable dehydrating agent such as $POCl_3$, $SOCl_2$, $P_2O_5$, toluene sulfonyl chloride in pyridine, oxalyl chloride in pyridine, etc. This reaction can be performed in the absence of or with an inert co-solvent such as xylene. The dehydrating agent such as $POCl_3$ is employed in equivalent amounts or greater and preferably in amounts of from about 2 to about 15 equivalents. Any suitable temperature and time can be employed for performing the reaction, but generally heat is added to speed up the reaction. Preferably, the reaction is performed at or near reflux.

The cyano compound of formula VI is reacted with a Grignard reagent (formula VII) prepared from the appropriate 1-(N-protected)-4-halopiperidine. Any suitable N-protecting group known in the art to protect the piperidinyl nitrogen atom from reaction during formation of the Grignard reagent of formula VI can be employed. The N-protecting group represented by $R^5$, can be any group which will allow the preparation of the Grignard reagent VII from the corresponding 4-halopiperidine without undesired side reactions. Suitable N-protecting groups include alkyl (e.g., methyl), aryl (e.g. phenyl or substituted phenyl), alkyloxyalkyl (e.g., methoxymethyl), benzyloxyalkyl (e.g., benzyloxymethyl), substituted benzyloxyalkyl (e.g. (di-p-methoxyphenyl)methyl), triphenylmethyl, tetrahydropyranyl, diphenyl posphinyl, benzene sulfenyl, etc. The N-protecting group can be later removed by conventional means.

The reaction between compounds of formulae VI and VII is generally performed in an inert solvent such as an ether, toluene or tetrahydrofuran. This reaction is performed under the general conditions for a Grignard reactions, e.g., at temperatures of from about 0°C to about 75°C. The resulting intermediate is hydrolyzed, e.g, with aqueous acid such as aqueous HCl, to prepare the corresponding ketone of formula II.

The process of this invention, i.e., the intramolecular condensation of a compound of formula II is performed by treating the compound II with a super acid having Hammett acidity function of minus 12, or lower e.g., minus l3, minus 14, etc. This measure of acidity is defined in Hammett, Louis P., and Deyrup, Alden J., Journal of the American Chemical Society, Vol. 54, 1932, p. 2721. Suitable super acids for this purpose include, for example, $HF/BF_3$, $CF_3SO_3H$, $CH_3SO_3H/BF_3$, $FSO_3H$ , etc. The reaction can be performed in the absence of or with a suitable inert co-solvent such as $CH_2Cl_2$. The temperature and time

of the reaction vary with the acid employed. For example, with $HF/BF_3$ as the super acid system the temperature may be controlled so as to minimize side reactions, such as HF addition to the double bond of the rings. For this purpose, the temperature is generally in the range of from about +5° to -50°C, preferably from about -30° to -35°C. With $CF_3SO_3H$ as the super acid system, the reaction may be run at elevated temperatures, e.g., from about 25° to about 150°C and at lower temperatures but the reaction then takes longer to complete.

Generally the super acid is employed in excess, preferably in amount of from about 1.5 to about 30 equivalents. For example, with $HF/BF_3$ as the super acid system the volume/weight ratio of HF to the compound of formula II in the reaction mixture is preferably from about 30 to about 1.5, more preferably 2.5 to 1.5. In such system, the weight/weight ratio of $BF_3$ to the compound of formula II in the reaction mixture is preferably from about 15 to about 0.75, more preferably from about 1 to about 0.75.

The N-protecting group represented by $R^{5'}$ can be converted to -H or $-COOR^7$ prior to or after the ring closure reaction by any conventional method. For example, an $R^{5'}$ methoxymethyl group may be converted to -H by treatment with boron trifluoride etherate, acetic anhydride and LiBr, while an $R^{5'}$ benzyloxymethyl group can be converted to -H by catalytic reduction followed by basic hydrolysis. The resulting compounds wherein $R^5$ is -H can be converted to compound wherein $R^5$ is $-COOR^7$ by reaction with a compound of the formula $ZCOOR^7$ wherein Z is chloro, bromo or iodo. Further, when $R^5$ is an alkyl group, such compounds may be directly converted to compounds wherein $R^5$ is $-COOR^7$ by reaction with a compounds $ZCOOR^7$ as described below. Examples of the latter two processes are disclosed in U.S. patent No. 4,282,233.

Alternatively, the compounds of formula I wherein $R^5$ is hydrogen can be prepared by dealkylation of a compound of formula I wherein $R^5$ is alkyl (preferably methyl), e.g., by reaction with cyanogen bromide and subsequent hydrolysis of the N-cyano product with, for example, aqueous acid solution.

The compounds of formula I can form salts with pharmaceutically acceptable acids such as hydrochloric, methanesulfonic, sulfuric, acetic, maleic, fumaric, phosphoric and the like. The salts are prepared by contacting the free base form with a sufficient amount of the desired acid to produce a salt in the conventional manner. The free base form may be regenerated by treating the salt forms with a base. For example, dilute aqueous base solutions may be utilized. Dilute aqueous sodium hydroxide, potassium carbonate, ammonia, and sodium bicarbonate solutions are suitable for this purpose. The free base form differs from the respective salt forms somewhat in certain physical properties such as solubilitly in polar solvents, but the salts are otherwise equivalent to the respective free base form for purposes of the invention.

The compounds of the invention and their corresponding salts can exist in unsolvated as well as solvated forms, including hydrated forms. In general, the solvated forms, with pharmaceutically acceptable solvents such as water, ethanol and the like are equivalent to the unsolvated forms for purposes of the invention.

Certain compounds of formula I are well known in the art, e.g. from U.S. patents Nos. 3,326,924, 3,717,647, 4,282,233 and 4,072,756, as pharmaceutically active compounds having antihistaminic and/or anti-allergy properties.

Within the scope of compounds defined by formula I the narrow group having the formula XIII

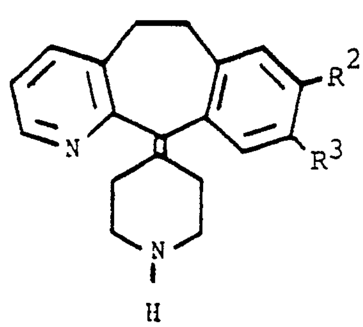

XIII

and salts thereof,

wherein $R^2$ is

F, Br or I;

and

$R^3$ is hydrogen, are novel compounds.

the following compounds with the general formula XIII also fall in the scope of the invention when $R^2$ and $R^3$ are combined as follows:

| | R2 | R3 |
|---|---|---|
| 1. | H | I |
| 2. | CF3 | H |
| 3. | F | F |
| 4. | Br | Br |
| 5. | I | I |
| 6. | CF3 | CF3 |
| 7. | F | Cl |

They have not been specifically described in the literature and have been found to have superior properties compared to specific compounds described in the art.

The pharmaceutical compositions of this invention may be either solid or liquid. Solid form preparations include powders, tablets, dispersible granules, capsules, cachets and suppositories. Usually the active ingredient is admixed with one or more suitable carriers which may also act as diluents, flavoring agents, solubilizers, lubricants, suspending agents, binders or tablet disintegrating agents; it can also be an encapsulating material. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by adding the active component in water and may include suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins, methylcellulose, sodium carboxymethylcellulose and other well-known suspending agents.

The composition of the invention may also be deliverable transdermally, e.g., with a transdermally acceptable carrier. The transdermal compositions can take the form of creams, lotions and/or emulsions, can be included in an appropriate adhesive for application to the skin or can be included in a transdermal patch of the matrix or reservoir type as are conventional in the art for this purpose.

Preferably, the transdermally acceptable composition is utilized to prepare a "reservoir type" or "matrix type" patch which is applied to the skin and worn for a specific period of time to permit the penetration of a desired amount of a compound of formula I through the skin. Most preferably, the patch of the invention will be worn for a period of about 24 hours and provide a total daily dosage of about 1 mg to about 40 mg, preferably from about 5 mg to about 10 mg, of a compound of the invention. The patch may then be replaced if necessary with a fresh patch, thereby providing a constant blood level of a compound of formula I to the patient in need thereof.

The utilization of this new transdermal dosage form and its prescribed regimen will provide the advantages described above. Other frequencies of dosage application are anticipated, for example, a once every 3 day frequency or a once every 7 day frequency. Although a once a day dosage regimen may be preferred, it is not intended that the invention be limited to any particular regimen.

The quantity of active compound in a unit dose of preparation may be varied or adjusted from 1 mg to 1000 mg according to the particular application. The compositions can, if desired, also contain other therapeutic agents, such as decongestants.

The dosages may be varied depending upon the requirements of the patient and the severity of the condition being treated. Determination of the proper dosage for a particular situation is within the skill of the art. For convenience, the total daily dosage may be divided and administered in portions during the day if desired.

The compounds of the invention possess antihistaminic properties. The antihistaminic properties of these compounds may be demonstrated by use of standard pharmacological testing procedures. For example, the ability of the compounds to reduce histamine-induced paw edema in mice may be measured by use of the following method.

Male $CF_1$ mice, 25-30 g, are housed under conditions of controlled temperature and humidity with a 12

hour dark/light cycle. Food and water are allowed ad libitum. The mice are randomly assigned to the treatment groups. One hour after treatment with a compound of the invention or vehicle, the mice are lightly anesthetized with ether. The left hind paw of each mouse serves as a control and is injected with 25 µl of isotonic saline. The right hind paw serves as the experimental paw and is injected with 25 µl of isotonic saline containing 13 µg histamine dihydrochloride. Thirty minutes later the mice are killed by cervical dislocation and both hind paws of each mouse are removed by cutting at the tarsal joint. The weight of each paw is recorded and the difference in weight between the compound-treated and the placebotreated groups is evaluated using Student's "t" test. The $ED_{50}$ values (the dose causing 50% inhibition of histamine-induced edema) and 95% confidence limits are determined by the Linear Least Square Dose-Response Method (Brownlee, K.A., "Statistical Theory and Methodology In Science and Engineering", 2nd Ed., J. Wiley and Sons, New York, 1965, pp. 346-349). As mentioned above, the novel compounds of this invention are potent antihistaminic agents without showing the usual CNS activity which is indicative of drowsiness.

Accordingly the compounds were also tested for antihistaminic activity by the procedures set forth in paragraph A below and for CNS activity by the procedures set forth in paragraphs B, C, and D below.

A. Prevention of histamine-induced lethality in guinea pigs. The compounds were evaluated for their ability to protect female albino guinea pigs (250-350 g) against death induced by the intravenous injection of histamine dihydrochloride at 1.1 mg/kg, which is approximately twice the $LD_{99}$. Doses of the antagonists were administered orally to separate groups of fasted animals 1 hour prior to the challenge with histamine and protection from death recorded for 30 minutes after histamine. $ED_{50}$ values were determined for each drug by prohibit analysis.

B. Antagonism of Physostigmine Lethality. The physostigmine-induced lethality test used was a modification of the technique reported by COLLIER et al., Br. J. Pharmac., 32, 295-310 (1968). Physostigmine salicylate (1.0 mg/kg s.c.) produces 100% lethality when administered to mice grouped 10 per plastic cage (11 x 26 x 13 cm). Test agents were administered orally 30 minutes prior to physostigmine. The number of survivors were counted 20 minutes after physostigmine administration.

C. Antagonism of Acetic Acid Writhing. The acetic acid writhing test used was essentially that described by HENDERSHOT and FORSAITH, J. Pharmac. Exp. Ther., 125, 237-240 (1959(, except that acetic acid rather than phenylguinone was used to elicit writhing. Mice were injected with 0.6% aqueous acetic acid at 10 mg/kg i.p. 15 minutes after oral administration of the test drug. The number of writhes for each animal was counted during a 10 minute period starting 3 minutes after acetic acid treatment. A writhe was defined as a sequence of arching of the back, pelvic rotation and hind limb extension.

D. Antagonism of Electro-Convulsive Shock (ECS). For the ECS test, a modification of the method of TOMAN et al., J. Neurophysiol., 9, 231-239 (1946), was used. One hour after oral administration of the test drug or vehicle, mice were administered a 13 mA, 50 cycle a.c. electroconvulsant shock (ECS) for 0.2 seconds via corneal electrodes. This shock intensity produces tonic convulsions, defined as extension of the hind limbs, in at least 95% of vehicle-treated, mice.

Of the above test procedures for measuring CNS activity of antihistamines, the physostigmine-induced lethality test is believed to be a major index of non-sedating characteristics, since it reflects mainly central anticholinergic potency which is believed to contribute to sedative activity.

The results from the above test procedures are set forth in the Table below:

Compounds tested were:

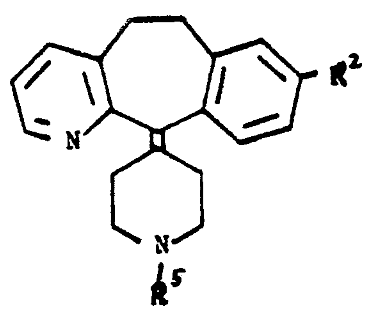

Compound A: $R^2$=Cl; $R^5$=H

Compound B: $R^2$=H; $R^5$=H

Compound C: $R^2$=Cl; $R^5$=$CH_3$

Compound D: $R^2$=H; $R^5$=$CH_3$

Compound E: $R^2$=F; $R^5$=H

Compound E is the preferred compound of those claimed herein. Compounds A to D are known compounds, whereby compound A is already known to be relatively non-sedating. (See European Patent Application No. 85101486).

TABLE

| Compound | Antihistaminic Activity | CNS Activity | | |
|---|---|---|---|---|
| | A. G. pig p.o. ED50 (mg/kg) | C. Phsostigmine lethality ED50 (mg/kg) | D. Acetic writhing ED50 (mg/kg) | E. ECS test ED50 (mg/kg) |
| A | 0.15 | 320 | 147 | 160 |
| B | 0.26 | 7.1 | 285.1 | 160 |
| C | 0.026 | 6 | 14.1 | 160 |
| D | 0.009 | 6.1 | 8.9 | 80 |
| E | 0.09 | 320 | 320 | 320 |

The above results demonstrate that the compounds of the invention are a potent antihistamines having low CNS activity indicative of non-sedation.

From the above test results, it may be concluded that the compounds of the invention would be essentially non-sedating at a clinically useful antihistamic dosage. Compound E (i.e., the 8-fluoro compound) is particularly preferred because it has also shown very low toxicity.

The amount and frequency of administration of the compounds of the invention and the pharmaceutically acceptable salts thereof will be regulated according to the judgment of the attending clinican considering such factors as age, condition and size of the patient as well as severity of the symptom being treated. A typical recommended dosage regimen is oral administration of from 5 to 100 mg/day, preferably l0 to 20 mg/day, in two to four divided doses to achieve relief of the symptoms.

The following examples are intended to illustrate, but not to limit, the present invention.

EXAMPLE I

A. N-(1,1-DIMETHYLETHYL)-3-METHYL-2-PYRIDINE CARBOXAMIDE

2-cyano-3-methyl pyridine (400 g) is suspended in t-BuOH (800 mL) and the mixture heated to 70° C. Concentrated sulphuric acid (400 mL) is added dropwise over 45 minutes. The reaction is complete after a further 30 minutes at 75° C. The mixture is then diluted with water (400 mL), charged with toluene (600 mL) and brought to pH 10 with concentrated aqueous ammonia. The temperature is kept at 50-55° C during the work up. The toluene phase is separated, the aqueous layer re-extracted and the combined toluene phases are washed with water. Removal of the toluene yields an oil, N (l,1-dimethylethyl)-3-methyl-2-pyridine carboxamide, from which solid product may crystallize. Product yield of 97% is determined by internal standard assay on gas chromatograph.

B. 3-[2-(3-FLUOROPHENYL)ETHYL]-N-(1,1-DIMETHYLETHYL)-2-PYRIDINE CARBOXAMIDE

Tetrahydrofuran (125 mL) and N-(1,1-dimethylethyl)-3-methyl-2-pyridine carboxamide (1 equivalent) are charged and cooled to -40° C under nitrogen. Two equivalents of n-butyllithium are then added over 40 minutes. When half the n-butyllithium is added the mixture turns purple. Sodium bromide (1.3 g) is added and then 3-fluoro-benzyl bromide (1.05 equivalents) is added dropwise (1:1 solution in tetrahydrofuran) over 40-50 minutes while the temperature is maintained at -40° C. After 30 minutes at -40° C, the mixture is diluted with water (250 mL) and the organic phase separated. This phase is dried over sodium sulphate and the solvent removed yielding an oil from which solid product, 3-[2-(3-fluorophenyl)ethyl]-N -(1,1-dimethylethyl)-2-pyridine carboxamide, may crystallize.

C. 3-[2-(3-FLUOROPHENYL)ETHYL]-2-PYRIDINE-CARBONITRILE

A solution of 3-[2-(3-fluorophenyl)ethyl]-N -(1,1-dimethylethyl)-2-pyridine carboxamide (36.4 g, 0.121 mole) in 123 mL (202.3 g, 1.32 mole) of phosphorous oxychloride is heated at 110° C. for 3.5 hours and stirred at ambient temperature an additional 15 hours. The reaction is quenched with ice and water and the pH of the solution is brought to 8 by the addition of a saturated aqueous solution of potassium carbonate. The product is extracted into ethyl acetate and the solution is concentrated to a residue. Following purification by silica gel chromatography and trituration with pentane, 16.2 g (0.072 mole) of product is obtained in 60% yield.

D. (1-METHYL-4-PIPERIDINYL)[3-[2-(3-FLUOROPHENYL)ETHYL]-2-PYRIDINYL]METHANONE

To a solution of 3-[2-(3-fluorophenyl)ethyl]-2-pyridine carbonitrile (28.0 g, 0.123 mole) in 150 mL of dry THF is added 92 mL (1.48 moles/liter, 0.136 mole) of N-methylpiperidyl magnesium chloride over 10 minutes maintaining the temperature at 45-50° C. The reaction is maintained at 40° C to 50° C for another 10 minutes and at ambient temperature for 45 minutes. The reaction is quenched to below pH 2 and aqueous hydrochloric acid and the resulting solution is stirred at 25° C for 1 hour. The pH of the solution is adjusted to about 8, the product is extracted with ethylacetate, and the solution is concentrated to a residue. Following purification by silica gel chromatography, 38.3 g of product is obtained as a brown oil.

E. 8-FLUORO-11-(1-METHYL-4-PIPERIDYLIDENE)-6-11-DIHYDRO-5H-BENZO[5,6]CYCLOHEPTA[1,2-b]-PYRIDINE

A solution of (1-methyl-4-piperidinyl)[3[2-(3-fluorophenyl)ethyl]-2-pyridinyl]methanone (15.0 g, 0.046 mole) in 74 mL (125.5 g, 0.837 mole) of trifluoromethane-sulfonic acid is stirred at ambient temperature for 18 hours. The reaction is quenched with ice-water and the solution made basic with potassium hydroxide. The product is extracted into ethyl acetate. The ethyl acetate solution is filtered to remove insolubles and the filtrate is concentrated to a residue. Following purification by silica gel chromatography, 5.4 9 (0.0175 mole) of product is obtained in 38% yield.

F. 8-FLUORO-11(1-ETHOXYCARBONYL-4-PIPERIDYLIDENE)-6,11-DIHYDRO-5H-BENZO[5,6]-CYCLOHEPTA[1,2-b]PYRIDINE

To a stirred solution of 8-fluoro-11-(1-methyl-4-piperidylidene)-6,1l-dihydro-5 H -benzo[5,6]cyclohepta-[1,2-b]pyridine (10.5 g, 34 mmol) and triethylamine (5.2 g, 52 mmol) in dry toluene (120 mL) at 80° C under an argon atmosphere, was added ethylchloroformate (18.5 g, 170 mmol) via a syringe. The reaction mixture was allowed to stir at this temperature for 30 minutes and at room temperature for one hour. The reaction mixture was then filtered and solvent was removed. The residue was triturated with pentane to give 10.1 grams (yield = 81%) of the title compound, m.p. = 116-118° C.

G. 8-FLUORO-11-(4-PIPERIDYLIDENE)-6,11-DIHYDRO-5H-BENZO-[5,6]CYCLOHEPTA[1,2-b]PYRIDINE

8-Fluoro-11-(1-ethoxycarbonyl-4-piperidylidene)-6,11-dihydro-5 H -benzo[5,6]cyclohepta[1,2-b]pyridine (3.6 g, 9.8 mmol.) was refluxed with KOH (4.5 g) in 50 mL of ethanol/water (1:1) for 66 hours under an argon atmosphere. The reaction mixture was poured into a brine solution and extracted twice with ethyl acetate. The extracts were combined and then dried over $Na_2SO_4$ and filtered. Solvent was removed to give 2.76 grams (yield = 95%) of the title compound, m.p. = 133.5-134.5° C.

EXAMPLE II

A. 3-[2-(3-FLUOROPHENYL)ETHENYL]-PICOLINAMIDE

A solution of 2-cyano-3-picoline (142 g, 1.2 mole) and 3-fluorobenzaldehyde (164 g,1.32 mole) in 750 mL of dry tetrahydrofuran (THF) is prepared. This solution is added dropwise to a solution of 162.0 grams

of potassium t-butoxide (1.44 mole) dissolved in 750 mL of dry THF at -15° C to -20° C.

The addition requires 1/2 hour, and the temperature is maintained below -15° C. The mixture is stirred at -15 to -20° C for 1 hour, then at 0 to 5° C for 2 hours.

Saturated $NH_4Cl$ solution (400 mL) is added to the mixture followed by 250 mL of $H_2O$. The mixture is stirred for 1/2 hour, and the aqueous layer is separated and extracted with 300 mL of $CH_2Cl_2$, which is combined with the THF layer. The organic solution is washed with 400 mL of saturated $NH_4Cl$ solution, dried over $Na_2SO_4$, treated with charcol and filtered through diatomaceous earth.

The solvent is removed on a rotary evaporator, and the oily residue is dissolved in 350 mL of boiling toluene. The mixture is filtered hot to remove impurities and cooled overnight at 0 to 5° C. The off-white solid that precipitates is filtered, washed twice with 100 mL of ccld toluene, and dried at 60° C for 6 hours, yielding 122.1 grams (42.1%) of the title compound, m.p. = 153-155° C.

B. 3-[2-(3-FLUOROPHENYL)ETHYL]-PICOLINAMIDE

A solution is prepared by dissolving 121 grams of 3-[2-(3-fluorophenyl)ethenyl]-picolinamide (0.5 mole) in 500 mL of acetic acid. To the solution is added 8.0 grams of 5% Pd/C and the mixture is placed on a Parr hydrogenator overnight. A theoretical amount of $H_2$ is absorbed, and mixture is filtered through diatomaceous earth and poured into 4 liters of $H_2O$. The off-white suspension is stirred for 2 hours and cooled at 0 to 5° C for 20 hours.

The solid product 3-[2-(3-fluorophenyl)ethyl]-picolinamide is filtered and washed three times with 100 mL of $H_2O$ and dried at 60° C for 20 hours, yielding 108 grams (yield = 88.6%) of the title compound, m.p. = 102-104° C.

C. 3-[2-(3-FLUOROPHENYL)ETHYL]-PICOLINIC ACID

A suspension of 73.2 grams of 3-[2-(3-fluorophenyl)ethyl]-picolinamide (0.3 mole) in 500.mL of ethanol and 125 grams of 45% KOH (1.0 mole) is prepared. $H_2O$ (200 mL) is added, and the mixture is refluxed for 20 hours. TLC shows complete conversion to the acid.

The alcohol is removed by distillation until the vapor temperature reaches 100° C. The suspension is cooled to room temperature, 100 mL of $H_2O$ is added and the solution is brought to a pH of 4-4.5 with 12N HCl (110 mL). The suspension is stirred for 1 hour and cooled overnight. The solid is filtered, washed three times with 100 mL of $H_2O$ and dried at 65° C for 24 hours, yielding 69.6 grams (yield = 94.8%) of the title compound, m.p. = 118-122° C.

D. 8-FLUORO-6,11-DIHYDRO-5H-BENZO-[5,6]-CYCLOHEPTA[1,2-b]PYRIDIN-11-ONE

A solution of 61.3 grams of 3-[2-(3-fluorophenyl)ethyl]-picolinic acid (0.25 mole) in 900 mL of tetrachloroethane is prepared. Anhydrous HCl gas is passed through the solution at room temperature for 1 1/2 hours. Oxalylchloride (48.3 grams, 0.38 mole) is carefully added, and stirred for 24-26 hours at room temperature (slight heating at 35-40° C for 4 hours is needed to obtain a dark solution). The solution is cooled to 0-5° C, and 67 grams of $AlCl_3$ (0.5 mole) are slowly added over 1/2 hour. The mixture is stirred at 0 to 5° C for 18 hours. An additional 17 grams of $AlCl_3$(0.125 mole) is added, and mixture is stirred for 2 hours.

Then 500 mL of 3.7% HCl is added, and the mixture is stirred for 1/4 hour and filtered through diatomaceous earth. The top aqueous layer is separated, and the organic layer is washed twice with 500 mL of 3.7 N HCl. The combined aqueous layer is washed twice with 500 mL of ether.

Benzene (1 liter) is added. The mixture is cooled to 5-10° C and brought to pH > 9 with slow addition of 390 grams of 50% NaOH . The mixture is stirred for 1/2 hour and filtered through diatomaceous earth. The aqueous layer is separated and washed twice with 300 mL of benzene, which are combined with the first benzene layer.

The combined organic layers are washed with 250 mL of 5% $NaHCO_3$ and 250 mL of saturated NaCl solution. The organic layer is dried over $Na_2SO_4$, and solvent is removed leaving 49.6 grams of a yellow solid. The solid is dissolved in 100 mL of butyl acetate (hot) and cooled for 24-48 hours at 0 to 5° C. The solid is filtered, washed twice with 30 mL of cold ethyl acetate, and dried at 60° C. for 20 hours, yielding 38.8 grams (yield = 68.5%) of the title compound, m.p. = 116-119° C.

E. 1-METHYL-4-[8-FLUORO-6,11-DIHYDRO-5H-BENZO-[5,6]CYCLOHEPTA[1,2-b]PYRIDIN-11-YL]-PIPERIDIN-11-OL

A suspension is prepared by mixing 22.7 grams of 11-oxo-8-fluoro-6,11-dihydro-5 H -benzo[5,6]-cyclohepta[1,2-b]pyridine (0.1 mole) with 500 mL of liquid $NH_3$, and 5.1 grams of Na(.22 mole) are added. The resulting blue mixture is stirred for 3/4 hour. A solution containing 15.9 grams of 4-chloro-N-methylpiperidine (0.12 mole) in 400 mL of dry THF is slowly added over 1/2 hour and stirred for 2 hours at -25° C.

$NH_4Cl$ (17.5 grams, .33 mole) is added, and the mixture is stirred for 1/2 hour until the mixture warms to 0° C. Saturated $NH_4Cl$ solution (200 mL) is added, followed by 50 mL of $H_2O$, and mixture is stirred for 1/4 hour.

The aqueous layer is separated and extracted with 200 mL of $CH_2Cl_2$, which is combined with the THF layer. The combined organic layer is washed with 250 mL of saturated $NH_4Cl$ solution, and dried over $Na_2SO_4$. The solvent is removed, leaving 34.7 grams of an oil, which crystallizes upon cooling.

The solid material is dissolved in 65 mL of hot n-butyl acetate and cooled overnight. The solid obtained is filtered, washed twice with 15 mL of cold ethyl acetate, and then dried at 75° C for 6 hours, yielding 15.8 grams of the title compound, m.p. = 123.5-125° C.

The solvent is removed from the filtrate, leaving 11.0 grams of a yellow solid, which is dissolved in 20 mL boiling ethyl acetate, filtered hot, and cooled at 0-5° C for 4 hours. The solid obtained is filtered, washed twice with 5 mL of ethyl acetate, and dried at 60° C for 5 hours, yielding 4.3 grams (total yield = 61.7%).

F. 8-FLUORO-11-(1-METHYL-4-PIPERIDYLIDENE)-6,11-DIHYDRO-5H-BENZO[5,6]CYLOHEPTA[1,2-b]-PYRIDINE

A solution of 13.1 grams of 1-methyl-4-[8-fluoro-6,11-dihydro-11-hydroxy-5 H -benzo-[5,6]cyclohepta-[1,2-b]pyridin-11-yl]piperidine (0.04 mole) in 40 mL of 93% $H_2SO_4$ is prepared. The solution is stirred overnight at room temperature. $CH_2Cl_2$ (200 mL) is added, and with external cooling, the mixture is neutralized to pH > 9 using 50% NaOH , while maintaining the temperature below 30° C. The aqueous layer is separated and re-extracted twice with 150 mL of $CH_2Cl_2$, which is combined with the first layer. The combined organic layer is washed with 150 mL of saturated $NaHCO_3$ solution and 150 mL of saturated NaCl solution, dried over $Na_2SO_4$, treated with charcoal and filtered through diatomaceous earth. The solvent is removed on a rotary evaporator leaving 12.9 grams of a yellow oil, which solidified upon standing.

The oil is dissolved in 70 mL of hot diisopropyl ether (6 parts) and poured into a hot solution of 9.5 grams of maleic acid (0.082 mole) dissolved in 60 mL of diisopropyl ether. The solution is cooled to 0-5° C with stirring and a yellow oil forms. The mixture is cooled overnight at 0-5° C with a yellow solid forming. The solid is filtered, washed twice with 20 mL of cold diisopropyl ether, and dried at 60° C for 4 hours, yielding 18.2 grams.

This solid is dissolved in 2 parts boiling diisopropyl ether and filtered hot. The filtrate is cooled at 0-5° C with stirring for 1 hour with a heavy white precipitate forming. The suspension is cooled at 0-5° C for 6 hours. The solid formed is filtered, washed three times with 15 mL of cold diisopropyl ether and dried at 75-80° C for 48 hours, yielding 15.6 grams (yield = 72.2 %) of the title compound, m.p. = 151-152° C.

The product of step F above can then be employed in the process described in Example 1.F. and 1.G. to provide 8-fluoro-11-(1-ethoxycarbonyl-4-piperidylidene)-6,11-dihydro-5 H -benzo[5,6]cyclohepta[1,2-b]-pyridine and 8-fluoro-11-(4-piperidylidene)-6,11-dihydro-5 H -benzo[5,6]cyclohepta[1,2-b]pyridine, respectively.

EXAMPLE III

8-CHLORO-6,11-DIHYDRO-11-(4-PIPERIDYLIDENE)-5H-BENZO[5,6]CYCLOHEPTA[l,2-b]PYRIDINE ACETIC ACID SALT

To 12 grams of sodium hydroxide in 30 mL ethyl alcohol (70%) add 6 grams of 8-chloro-6,11-dihydro-11-(1-ethoxy-carbonyl-4-piperidylidene)-5 H -benzo[5,6]-cyclohepta[1,2-b]pyridine (prepared as described in U.S. patent 4,282,233) and reflux with stirring for 24 hours. After about the first 6-8 hours an additional 30 mL of 70% ethyl alcohol may be added.

Remove about 50% of the solvent by distillation in vacuo. Add a small amount of ice water and acidify with glacial acetic acid.

Extract with chloroform (6-8x), since the product precipitates from the acetic acid solution as a thick

emulsion which cannot be filtered.

Concentrate the chloroform extracts to a small volume and precipitate the product with hexane. Crude m.p. 197-200° C.

Recrystallize from benzene-hexane to obtain the product, m.p. 199-200° C. Yield 4.0-4.5 grams.

EXAMPLE IV

A. 3-[2-(3-CHLOROPHENYL)ETHYL]-N-(1,1-DIMETHYLETHYL)-2-PYRIDINE CARBOXAMIDE

31.5 g of N-(1,1-dimethylethyl)-3-methyl-2-pyridine carboxamide is dissolved in 600 mL of dry tetrahydrofuran and the resulting solution is cooled to -40° C. Two equivalents of n-butyllithium in hexane are added while the temperature is maintained at -40° C. The solution turned deep purple-red. 1.6g of sodium bromide is added and the mixture is stirred. A solution of 26.5 g (0.174 mole) m-chlorobenzylchloride in 125 mL of tetrahydrofuran is added while the temperature is maintained at -40° C. The reaction mixture is stirred until the reaction is complete as determined by thin layer chromatography. Water is added to the reaction until the color is dissipated. The reaction mixture is extracted with ethyl acetate, washed with water, and concentrated to a residue. A yield of 92% for the product is shown by chromatography.

B. 3-[2-(3-CHLOROPHENYL)ETHYL]-2-PYRIDINE-CARBONITRILE

A solution of 3-[2-(3-chlorophenyl)ethyl]-N-(1,1-dimethylethyl)-2-pyridine carboxamide (175 g, 0.554 mole), in 525 mL (863 9, 5.63 mole) of phosphorous oxychloride is heated at reflux for 3 hours. Completion of the reaction is determined by thin layer chromatography. Excess phosphorous oxychloride is removed by distillation at reduced pressure and the residue is quenched into a mixture of water and isopropanol. The pH is brought to 5-7 by addition of 50% aqueous sodium hydroxide solution while maintaining the temperature below 30° C. The crystalline slurry of crude product is filtered and washed with water. Crude product is purified by slurrying the wet cake in hot isopropanol followed by cooling at 0-5° C. The product is filtered, washed with hexane and dried at below 50° C. Yield: 118g (HPLC purity 95.7%), m.p. 72°-73° C, 89.4% of theory.

C. (1-METHYL-4-PIPERIDINYL)[3-[2-(3-CHLOROPHENYL)ETHYL]-2-PYRIDINYL]METHANONE HYDROCHLORIDE

To a solution of product of Step B above (118 g, 0.487 mole) in 1.2 L of dry tetrahydrofuran is added 395 mL (2.48 mole/liter, 0.585 mole, 1.2 eg.) of N-methyl-piperidyl magnesium chloride over about 15 minutes maintaining the temperature at 45° C-50° C by cooling with water as necessary. The reaction is maintained at 40° C to 50° C for about another 30 minutes. Completion of the reaction is determined by thin-layer chromatography. The reaction is quenched to pH below 2 with 2N hydrochloric acid and the resulting solution is stirred at about 25° C for 1 hour. The bulk of the tetrahydrofuran is removed by distillation and the resulting solution is adjusted to pH 3.5 by the addition of aqueous sodium hydroxide. After cooling to 0 to 5° C, the crystalline hydrochloride salt product is filtered off, washed with ice cold water and dried to constant weight at 60° C. Yield: 168.2g (HALC purity 94%), m.p. 183°-185° C, 89% of theory.

D. 8-CHLORO-6,11-DIHYDRO-11-(1-METHYL-4-PIPERIDYLIDENE)-5H-BENZO[5,6]CYCLOHEPTA[1,2-b]-PYRIDINE

To a solution of a product of Step C above (59 g, 0.15 mole) in 120 mL (120 g, 6.0 mole) of hydrofluoric acid at -35° C is added boron trifluoridine (44.3 g, 0.66 mole) over 1 hour. Completeness of the reaction is determined by thin-layer chromatography. The reaction is quenched using ice, water and potassium hydroxide to a final pH of 10. The product is extracted into toluene and the toluene solution is washed with water and brine. The toluene solution is concentrated to a residue, which is dissolved in hot hexane. Insolubles are removed by filtration and the filtrate is concentrated to an off-white powder. Yield: 45.7 g (HPLC purity: 96%), 92% of theory.

Alternative Step D: 8-CHLORO-6,11-DIHYDRO-11-(1-METHYL-4-PIPERIDYLIDENE)-5H-BENZO[5,6]-CYCLOHEPTA[1,2-b]-PYRIDINE

A solution of 177 g (0.49 mole) of a product of Step C above in 480 mL (814.1 g, 5.31 mole) of trifluoromethanesulfonic acid at 90-95° C for 18 hours under nitrogen. Completeness of the reaction is determined by thin-layer chromatography. The cooled reaction is quenched with ice-water and the pH is adjusted to 6 with barium carbonate. The product is extracted into methylene chloride, which is concentrated under reduced pressure to about 1 liter and washed with water. The product is extracted into 1 N hydrochloric acid, which is treated with 30 g of Darco, and filtered through celite. The pH of the filtrate is adjusted to 10 with 50% aqueous sodium hydroxide and the product is extracted into methylene chloride, which is removed under reduced pressure. The residue is dissolved in hot hexane, which is filtered to remove insolubles. The filtrate is concentrated to a residual beige powder. Yield: 126 G (HPLC purity 80%), 65% of theory.

EXAMPLE V

8-CHLORO-6,11-DIHYDRO-11-(4-PIPERIDYLIDENE)-5H-BENZO[5,6]CYCLOHEPTA[1,2-b]PYRIDINE

The acetic acid salt prepared in Example III is dissolved in a minimum amount of water and the solution is made basic with a dilute aqueous solution of potassium carbonate. A pink colored oil separates. Extract the organic material with chloroform, wash with water and remove the solvent. Triturate the residue with hexane. Recrystallize from a large volume of hexane after charcoal decolorization to obtain the product, m.p. 151-152° C.

EXAMPLE VI

8-CHLORO-6,11-DIHYDRO-11-(4-PIPERIDYLIDENE)-5H-BENZO[5,6]CYCLOHEPTA[1,2-b]PYRIDINE

A. 8-CHLORO-6,11-DIHYDRO-11-(1-CYANO-4-PIPERIDYLIDENE)-5H-BENZO[5,6]CYCLOHEPTA[1,2-b]-PYRIDINE.

Dissolve 16.2 grams (0.05 mole) of 8-chloro-6,11-dihydro-11-(1-methyl-4-piperidylidene)-5 H -benzo-[5,6]cyclohepta[1,2-b]pyridine (prepared by the methods described in U.S. patent 3,326,924) in 300 mL of dry benzene. To this solution, add slowly under nitrogen a solution of cyanogen bromide (6.4 g) dissolved in 75 mL of benzene. Allow this mixture to stir at room temperature overnight (approximately 20 hours).

Filter the solution, and concentrate the filtrate in vacuo to a small volume and precipitate the product by the addition of petroleum ether or hexane until precipitation is complete. Filter and recrystallize from ethanol/water to yield the product 15 grams (89%), m.p. 140-142° C.

Anal. Calcd. for $C_{20}H_{18}N_3Cl$ : C,71.53; H,5.40; N,12.51. Found, C,71.73; H,5.43; N,12.27.

B. 8-CHLORO-6,11-DIHYDRO-11-(4-PIPERIDYLIDENE)-5H-BENZO[5,6]CYCLOHEPTA[1,2-b]PYRIDINE.

A solution of 14 grams of the N-cyano compound from part A in 60 mL of concentrated hydrochloric acid, 600 mL of glacial acetic acid and 400 mL of water is refluxed with stirring for 20 hours. The solvents are removed in vacuo and the residue dissolved in water and neutralized with ammonium hydroxide. The material is extracted several times with chloroform, the chloroform extracts washed with water and concentrated to dryness, and the residue triturated with petroleum ether or hexane to yield 11.5 grams (93%) m.p. 149-151° C. After recrystallization from hexane, the product melts at 150-151° C.

Anal. Calcd. for $C_{19}H_{19}N_2Cl$ : C,73.42; H,6.16; N,9.01. Found: C,73.19; H,6.14; N,8.91.

EXAMPLE VII

Step A: N-(1,1-dimethylethyl)-3-methyl-2-pyridine carboxamide.

2-cyano-3-methyl pyridine (400g) is suspended in t-butyl alcohol (800 mL) and the mixture heated to 70° C. Concentrated sulphuric acid (400 mL) is added dropwise over 45 minutes. The reaction is complete after a further 30 minutes at 75° C. The mixture is then diluted with water (400 mL), charged with toluene (600 mL) and brought to pH 10 with concentrated aqueous ammonia. The temperature is kept at 50-55° C

during the work up. The toluene phase is separated, the aqueous layer re-extracted and the combined toluene phases are washed with water. Removal of the toluene yields an oil from which solid product may crystallize. Product yield of 97% determined by internal standard assay on gas chromatograph.

Step B: 3-[2-(phenyl)ethyl]-N-(1,1-dimethylethyl)-2-pyridine carboxamide.

Tetrahydrofuran (125 mL), N-(1,1-dimethylethyl)-3-methyl-2-pyridine carboxamide (1 equivalent) and sodium bromide (1.3 g) are charged and cooled to -40 °C under nitrogen. Two equivalents of n-butyllithium are then added over 40 minutes. When half the n-butyllithium is added the mixture turns purple. Benzyl chloride (1.05 equivalents) is then added dropwise (1:1 solution in tetrahydrofuran) over 40-50 minutes while the temperature is maintained at -40 °C. After 30 minutes at -40 °C, the mixture is diluted with water (250 ml) and the organic phase separated. This phase is dried over sodium sulphate and the solvent removed yielding an oil from which solid product may crystallize. Product yield of 94% determined by internal standard assay on gas chromatograph.

Step C: Cyclization to 5,6-dihydro-11H-benzo[5,6]cyclohepta[1,2-b]pyridin-11-one

Polyphosphoric acid (123.75g) and water (1.25 mL) are heated to 200 °C. 3-[2-(phenyl)ethyl]-N -(1,1-dimethylethyl)-2-pyridine carboxamide is then added. After 30 minutes at 200 °C the mixture is allowed to cool. Then the mixture is diluted with water and toluene is added. The mixture is brought to pH 10 with 40% aqueous NaOH. An internal standard assay on gas chromatograph determined a yield of 58% for 5,6-dihydro-11 H -benzo-[5,6]cyclohepta[1,2-b]pyridin-11-one. Crystallization of title compound from toluene-hexane gave m.p. 118.5-119.7 °C.

EXAMPLE VIII

Ethyl chloroformate (8.5 mL, 9.65 g, 0.089 mole) is slowly added at 80 °C to a solution of 8-fluoro-11-(1-methyl-4-piperidylidene)-6,11-dihydro-5 H -benzo[5,6]cyclohepta(1,2-b]pyridine (5.4 g, 0.0175, mole) and triethylamine (3.6 mL, 2.61 g, 0.026 mole) in 60 mL of toluene. Following complete addition, the temperature is maintained at 80 °C for 1 hour. The reaction mixture is treated with charcoal, filtered, and concentrated to a residue. Following purification by silica gel chromatography and crystallization from pentane, 4.10 g (0.011 mole) of 8-fluoro-6,11-dihydro-11-(1-ethoxycarbonyl-4-piperidylidene)-5 H -benzo-[15,6]-cyclohepta[1,2-b]pyridine is obtained in 63% yield.

By analogous procedures, the corresponding 8-chloro, 8-bromo, 8,9-dichloro, 9-chloro, and 9-fluoro analogs of this 8-fluoro-11-(1-ethoxycarbonyl-4-piperidylidene) compound can be prepared.

EXAMPLE IX

A solution of 8-fluoro-6,11-dihydro-11-(1-ethoxycarbonyl-4-piperidylidene)-5 H -benzo[5,6] cyclohepta-[1,2-b]pyridine (3.6 g, 0.0098 mole) and potassium hydroxide (4.5 g, 0.094 mole) in 50 mL of ethanol:water (1:1) is heated at reflux for 66 hours. The reaction mixture is diluted with brine and the product is extracted into ethyl acetate. The solution is concentrated to a residual solid which is washed with acetone/ethyl acetate to yield 2.76 g (0.0094 mole) of 8-fluoro-11-(4-piperidylidene)-6,11-dihydro-5H-benzo[5,6]-cyclohepta[1,2-b]pyridine.

By analogous procedures, the corresponding 8-chloro, 8-bromo, 8,9-dichloro, 9-chloro and 9-fluoro analogs of this 8-fluoro-11-(4-piperidylidene) compound can be prepared.

EXAMPLE X

A solution of (1-ethoxycarbonyl-4-piperidinyl)[3-[2-(3-chlorophenyl)ethyl]-2-pyridinyl]methanone hydrochloride (0.5 g, 1.25 mmol) (prepared by reacting the corresponding 1-methyl-H-piperidinyl compound with ethyl chloroformate) in 1.5 mL of trifluoromethane sufonic acid is stirred at ambient temperature for 24 hours. The reaction is diluted with ice and water, neutralized with barium carbonate, and the product

extracted into ethyl acetate. The solvent is removed and following purification of the residue by silica gel chromatography, 8-chloro-6,11-dihydro-11-(1-ethoxycarbonyl-4-piperidylidene)-5 H -benzo[5,6]-cyclohepta-[1,2-b]pyridine is obtained.

The compound 8-chloro-6,11-dihydro-11-(4-piperidylidene)-5 H -benzo[5,6]cyclohepta[1,2-b]pyridine can be prepared by the above method by substituting (4-piperidinyl)[3-[2-(3-chlorophenyl)ethyl]-2-pyridinyl] methanone in place of the (1-ethoxycarbonyl-4-piperidylidene)[3-[2-(3-chlorophenyl)ethyl]-2-pyridinyl]-methanone hydrochloride.

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

EXAMPLE XI

5,6-DIHYDRO-11H-BENZO[5,6]CYCLOHEPTA[1,2-B]-PYRIDIN-11-ONE

A solution of 0.50 g (1.57 mmol) of t-butylamide in 3 mL of trifluoromethanesulfonic acid was heated at 95°C under nitrogen for three days. The reaction mixture was poured into ice water and adjusted to pH 10 with 50% aqueous sodium hydroxide. The product was extracted with ethyl acetate and the organic layer was concentrated to give 0.85 g of residue. Column chromatography on silica gel afforded 0.28 g (73%) of azaketone as a light brown solid.

Typical examples of compounds of formula XIII obtainable by the process of this invention and claimed herein are

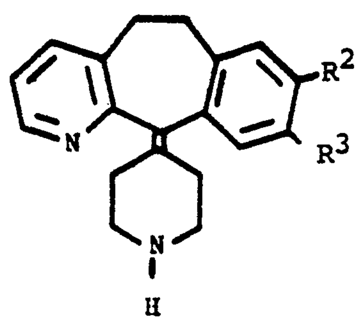

I

| Compound No. | $R^2$ | $R^3$ |
|---|---|---|
| 1 | H | Cl |
| 2 | H | Br |
| 3 | H | I |
| 4 | H | $CF_3$ |
| 5 | Br | H |
| 6 | I | H |
| 7 | $CF_3$ | H |
| 8 | F | F |
| 9 | Br | Br |
| 10 | I | I |
| 11 | $CF_3$ | $CF_3$ |
| 12 | F | Cl |

## Claims

**1.** Process for the preparation of compounds of the general formula I

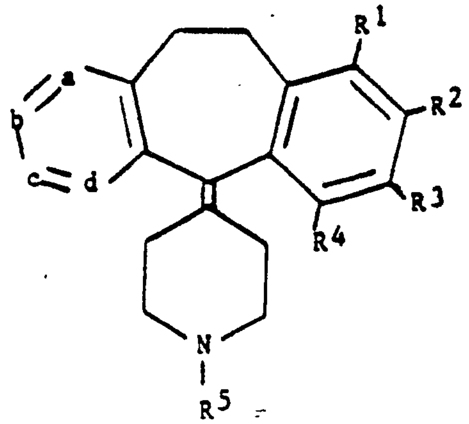

I

wherein a, b, c, and d represent CH or one of a, b, c and d represents N and the others represent CH;

$R^1$, $R^2$, $R^3$ and $R^4$ may be the same or different and each independently represents hydrogen, alkyl having from 1 to 6 carbon atoms, fluoro, chloro, bromo, iodo, nitro, alkoxy having from 1 to 6 carbon atoms or trifluoromethyl;

$R^5$ is methyl -H or $-COOR^7$, wherein $R^7$ represents $C_1$ to $C_{12}$ alkyl, substituted $C_1$ to $C_{12}$ alkyl, $C_2$ to $C_{12}$ alkenyl, substituted $C_2$ to $C_{12}$ alkenyl, phenyl, substituted phenyl, $C_7$ to $C_{10}$ phenylalkyl or $C_7$ to $C_{10}$ phenylalkyl wherein the phenyl moiety is substituted, or $R^7$ is 2-, 3- or 4-piperidyl or N-substituted piperidyl, wherein the substituents on said substituted $C_1$ to $C_{12}$ alkyl and on said substituted $C_2$ to $C_{12}$ alkenyl are selected from amino and substituted amino and the substituents on said substituted amino are selected from $C_1$ to $C_6$ alkyl, the substituents on said substituted phenyl and on said substituted phenyl moiety of the $C_7$ to $C_{10}$ phenylalkyl are selected from $C_1$ to $C_4$ alkyl and halo, and the substituent on said N-substituted piperidyl is $C_1$ to $C_4$ alkyl;

and pharmaceutically acceptable salts of such compounds,

whereby a compound of the general formula II

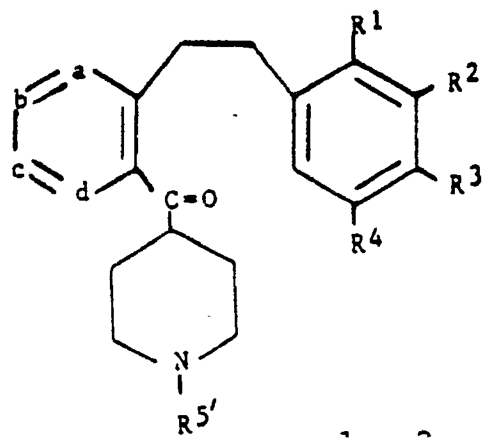

II

wherein a, b, c, d, $R^1$, $R^2$, $R^3$ and $R^4$ are as defined above, and

$R^{5'}$ is as defined for $R^5$ above or represents an N-protecting group, is subjected to an intramolecular condensation followed, if necessary or desired, by one or more of the following steps:

a) removal of any N-protecting group represented by $R^{5'}$;

b) transformation of a compound wherein $R^5$ is methyl or $-COOR^7$ into a compound wherein $R^5$ is hydrogen;

c) transformation of a compound wherein $R^5$ is hydrogen into a compound wherein $R^5$ is $-COOR^7$ or methyl.

d) transformation of a compound wherein $R^5$ is methyl into a compound wherein $R^5$ is $-COOR^7$; and

e) transformation of a compound of formula I obtained according to any one of the steps above into a pharmaceutically acceptable salt;

characterized in that the intramolecular condensation is performed by treating the compound of formula II with a super acid having a Hammett acidity function of minus 12 or lower.

**2.** Process according to Claim 1, characterized in that the acid has a Hammett acidity function of minus 13 or minus 14.

**3.** Process according to Claim 1 or Claim 2, characterized in that the acid used is selected from $HF/BF_3$, $CF_3SO_3H$, $CH_3SO_3H/BF_3$ and $FSO_3H$.

**4.** Compounds of the general formula XIII

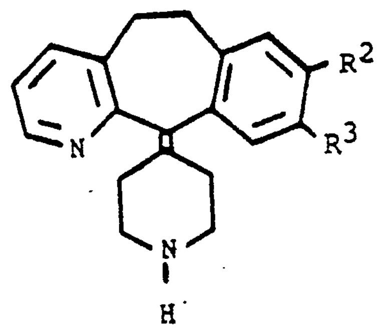

XIII

wherein R2 is F, Br or I and R3 is H; and pharmaceutically acceptable salts thereof, and further including the compounds wherein R2 and R3 can be any of the following combinations:

| | R2 | R3 |
|---|---|---|
| 1. | H | I |
| 2. | CF3 | H |
| 3. | F | F |
| 4. | Br | Br |
| 5. | I | I |
| 6. | CF3 | CF3 |
| 7. | F | Cl |

**5.** Compounds as claimed in claim 4 wherein $R^2$ is F and $R^3$ is H.

**6.** A pharmaceutical composition comprising, as active ingredient, a compound of formula XIII defined in claim 4 together with a pharmaceutically acceptable carrier.

**7.** The use of a compound as defined in claim 4 or claim 5 for the preparation of pharmaceutical compositions having anti-allergic activity.

CLAIMS FOR THE FOLLOWING CONTRACTING STATE: AT

1. Process for the preparation of compounds of the general formula I

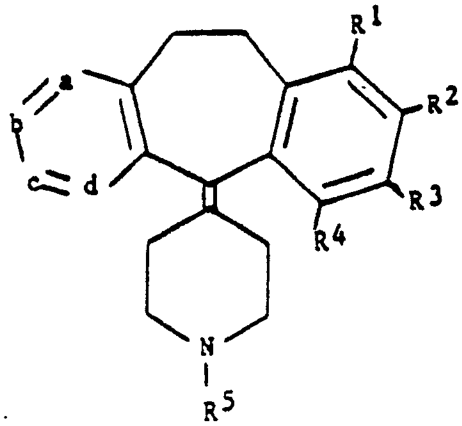

I

wherein a, b, c, and d represent CH or one of a, b, c and d represents N and the others represent CH;

$R^1$, $R^2$, $R^3$ and $R^4$ may be the same or different and each independently represents hydrogen, alkyl having from 1 to 6 carbon atoms, fluoro, ohloro, bromo, iodo, nitro, alkoxy having from 1 to 6 carbon atoms or trifluoromethyl;

$R^5$ is methyl, -H or -$COOR^7$, wherein $R^7$ represents $C_1$ to $C_{12}$ alkyl, substituted $C_1$ to $C_{12}$ alkyl, $C_2$ to $C_{12}$ alkenyl, substituted $C_2$ to $C_{12}$ alkenyl, phenyl, substituted phenyl, $C_7$ to $C_{10}$ phenylalkyl or $C_7$ to $C_{10}$ phenylalkyl wherein the phenyl moiety is substituted, or $R^7$ is 2-, 3- or 4-piperidyl or N-substituted piperidyl, wherein the substituents on said substituted $C_1$ to $C_{12}$ alkyl and on said substituted $C_2$ to $C_{12}$ alkenyl are selected from amino and substituted amino and the substituents on said substituted amino are selected from $C_1$ to $C_6$ alkyl, the substituents on said substituted phenyl and on said substituted phenyl moiety of the $C_7$ to $C_{10}$ phenylalkyl are selected from $C_1$ to $C_4$ alkyl and halo, and the substituent on said N-substituted piperidyl is $C_1$ to $C_4$ alkyl;

and pharmaceutically acceptable salts of such compounds,

whereby a compound of the general formula II

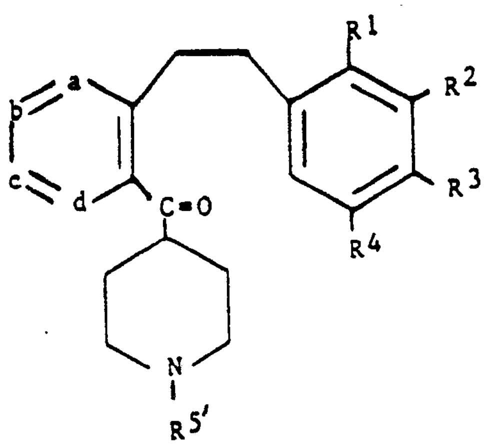

II

wherein a, b, c, d, $R^1$, $R^2$, $R^3$ and $R^4$ are as defined above, and

$R^{5'}$ is as defined for $R^5$ above or represents an N-protecting group, is subjected to an intramolecular condensation followed, if necessary or desired, by one or more of the following steps:

a) removal of any N-protecting group represented by $R^{5'}$;

b) transformation of a compound wherein $R^5$ is methyl or -$COOR^7$ into a compound wherein $R^5$ is hydrogen;

c) transformation of a compound wherein $R^5$ is hydrogen into a compound wherein $R^5$ is -C-$OOR^7$hydrogen into a compound wherein $R^5$ is -$COOR^7$ or methyl.

d) transformation of a compound wherein $R^5$ is methyl into a compound wherein $R^5$ is -$COOR^7$; and

e) transformation of a compound of formula I obtained according to any one of the steps above into a pharmaceutically acceptable salt;

characterized in that the intramolecular condensation is performed by treating the compound of formula II with a super acid having a Hammett acidity function of minus 12 or lower.

2. Process according to Claim 1, characterized in that the acid has a Hammett acidity function of minus 13 or minus 14, and in particular is selected from $HF/BF_3$, $CF_3SO_3H$, $CH_3SO_3H/BF_3$ and $FSO_3H$ .

3. Process for making compounds of the general formula XIII

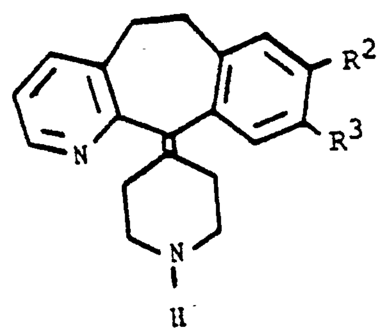

XIII

wherein $R^2$ is F, Br or I and $R^3$ is H;

and pharmaceutically acceptable salts thereof, characterized in that

A: a compound of the general formula

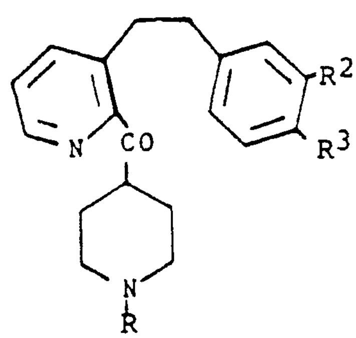

wherein $R^2$ and $R^3$ are as defined above and R is H or an N-protecting group, is subjected to an intramolecular condensation; or

B: a compound of the general formula

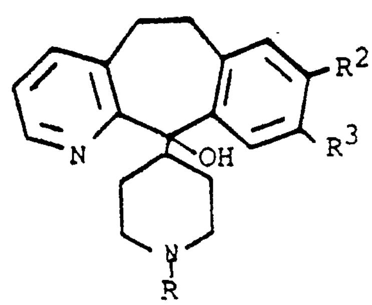

wherein $R^2$, $R^3$ and R are as defined above, is subjected to a dehydration introducing a double bond between the position 11 of the tricyclic nucleus and the piperidinyl residue;

followed, if necessary, by removal of any N-protecting group represented by R and, if desired, transformation into an acid addition salt.

4. Process according to claim 3 wherein $R^2$ and $R^3$ can be any of the following combinations:

| | $R^2$ | $R^3$ |
|---|---|---|
| 1. | H | I |
| 2. | $CF_3$ | H |
| 3. | F | F |
| 4. | Br | Br |
| 5. | I | I |
| 6. | $CF_3$ | $CF_3$ |
| 7. | F | Cl . |

**5.** Process according to Claim 3, wherein the intramolecular condensation is carried by means of an acid, preferably sulfuric acid.

**6.** Process according to Claim 4 wherein the acid used is a super acid as defined in Claim 1.

**7.** Process according to any one of Claims 1 to 6 wherein the N-protecting group represented by R is $C_1$-$C_4$-alkyl, preferably methyl.

**8.** Process for preparing a pharmaceutical composition, characterized in that a compound of formula XIII as defined in Claim 3 is brought into a form suitable for pharmaceutical application.

**9.** The use of a compound of formula XIII as defined in Claim 3 for the preparation of pharmaceutical compositions having anti-allergic activity.

**10.** A pharmaceutical composition comprising, as active ingredient, a compound of formula XIII defined in claim 3 together with a pharmaceutically acceptable carrier.

## Revendications

**1.** Procédé de préparation de composés de formule générale I :

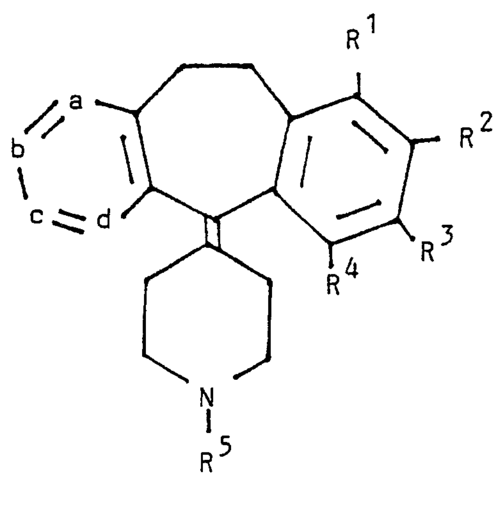

I

où a, b, c et d représentent CH ou un de a, b, c et d représente N et les autres représentent CH;

$R^1$ , $R^2$, $R^3$ et $R^4$ peuvent être identiques ou différents et chacun représente indépendamment de l'hydrogène, un alkyle ayant de 1 à 6 atomes de carbone, un fluoro, un chloro, un bromo, un iodo, un nitro, un alcoxy ayant de 1 à 6 atomes de carbone ou un trifluorométhyle;

$R^5$ est un méthyle, -H ou -COOR$^7$, où $R^7$ rerpésente un alkyle de $C_1$ à $C_{12}$, un alkyle de $C_1$ à $C_{12}$ substitué, un alcényle de $C_2$ à $C_{12}$, un alcényle de $C_2$ à $C_{12}$ substitué, un phényle, un phényle substitué, un phénylakyle de $C_7$ à $C_{10}$, ou un phénylalkyle de $C_7$ à $C_{10}$ où la moitié phényle est substituée, ou $R^7$ est une 2-, 3- ou 4-pipéridyle ou une pipéridyle N substituée, où les substituants sur ledit alkyle $C_1$-$C_{12}$ substitué et sur ledit alcényle $C_2$ à $C_{12}$ substitué sont choisis parmi un amino, un

amino substitué et les substituants dudit amino substitué sont choisis parmi l'alkyle de $C_1$ à $C_6$, les substituants dudit phényle substitué et de ladite moitié phényle substituée du phénylalkyle de $C_7$ à $C_{10}$ sont choisis parmi l'alkyle de $C_1$ à $C_4$ et l'halo, et le substituant de ladite pipéridyle N-substituée est un alkyle de $C_1$ à $C_4$;

et des sels acceptables pharmaceutiquement de ces composés,

de cette façon, un composé de la formule générale II

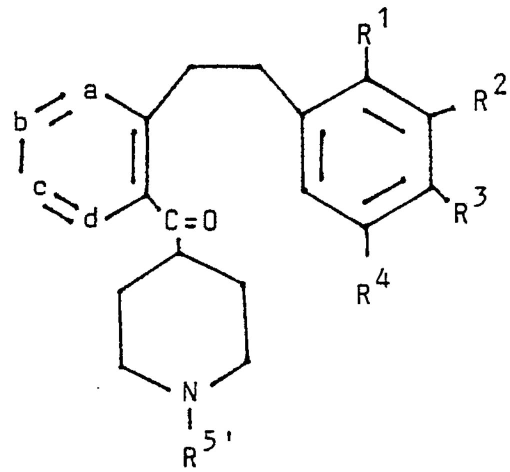

II

où a, b, c, d, $R^1$ , $R^2$, $R^3$ et $R^4$ sont tels que définis ci-dessus, et

$R^{5'}$ a la même définition que $R^5$ ci-dessus ou représente un groupe protégeant N, est soumis à une condensation intramoléculaire suivie, si nécessaire ou désiré, par une ou plusieurs des étapes suivantes :

a) extraction d'un groupe quelconque protégeant N, représenté par $R^{5'}$,

b) transformation d'un composé, où $R^5$ est un méthyle ou un $-COOR^7$ dans un composé, où $R^5$ est un hydrogène;

c) transformation d'un composé, où $R^5$ est un hydrogène dans un composé, où $R^5$ est $-COOR^7$ ou un méthyle.

d) transformation d'un composé, où $R^5$ est un méthyle dans un composé où $R^5$ est $-COOR^7$; et

e) transformation d'un composé de formule I obtenu selon l'une quelconque des étapes ci-dessus en un sel acceptable pharmaceutiquement; caractérisé en ce que la condensation intramoléculaire est réalisée par le traitement du composé de formule II, avec un super acide ayant une fonction d'acidité Hammett de moins de 12, ou plus basse.

2. Procédé selon la revendication 1, caractérisé en ce que l'acide a une fonction acide Hammett de moins de 13 ou moins de 14.

3. Procédé selon la revendication 1 ou la revendication 2, caractérisé en ce que l'acide utilisé est choisi parmi $HF/BF_3$, $CF_3SO_3H$, $CH_3SO_3H/BF_3$ et $FSO_3H$ .

4. Composés de formule générale XIII

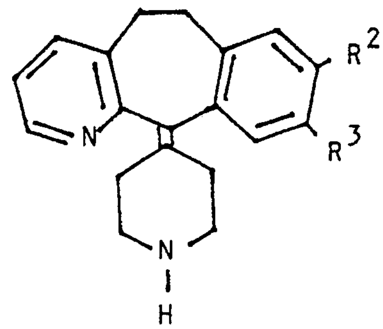

XIII

où $R^2$ est F, Br ou I et $R^3$ est H; et leurs sels acceptable s pharmaceutiquement et de plus incluant les composés où $R^2$ et $R^3$ peuvent être l'une des combinaisons suivantes :

| | $R^2$ | $R^3$ |
|---|---|---|
| 1. | H | I |
| 2. | $CF_3$ | H |
| 3. | F | F |
| 4. | Br | Br |
| 5. | I | I |
| 6. | $CF_3$ | $CF_3$ |
| 7. | F | Cl |

**5.** Composés selon la revendication 4, où $R^2$ est F et $R^3$ est H.

**6.** Composition pharmaceutique comprenant, comme ingrédient actif, un composé de formule XIII défini dans la revendication 4 avec un porteur acceptable pharmaceutiquement.

**7.** Utilisation d'un composé, tel que défini dans la revendication 4 ou 5, pour la préparation de compositions pharmaceutiques ayant une activité anti-allergique.

Revendications pour l'Etat contractant suivant: AT

**1.** Procédé pour la préparation de composés de formule générale I

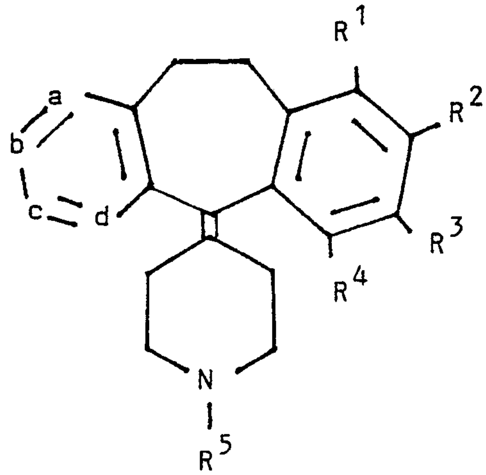

I

où a, b, c, et d représentent CH ou un de a, b, c et d représente N et les autres représentent CH;

$R^1$, $R^2$, $R^3$ et $R^4$ peuvent être identiques ou différents et chacun représente indépendamment un hydrogène, un alkyle ayant de 1 à 6 atomes de carbone, un fluoro, un chloro, un bromo, un iodo, un nitro, un alcoxy ayant de 1 à 6 atomes de carbone ou un trifluorométhyle;

$R^5$ est un méthyle, -H ou -$COOR^7$, où $R^7$ représente un alkyle de $C_1$ à $C_{12}$, un alkyle de $C_1$ à $C_{12}$ substitué, un alcényle de $C_2$ à $C_{12}$, un alcényle de $C_2$ à $C_{12}$ substitué, un phényle, un phényle substitué, un phénylalkyle de $C_7$ à $C_{10}$ ou un phénylalkyle de $C_7$ à $C_{10}$ où la moitié de phényle est substituée, ou $R^7$ est une 2-, 3- ou 4-pipéridyle ou une pipéridyle N substitué, où les substituants de ladite alkyle $C_1$ à $C_{12}$ substituée et ledit alcényle de $C_2$ à $C_{12}$ substitué sont choisis parmi un amino et un amino substitué et les substituants dudit amino substitué sont choisis parmi l'alkyle de $C_1$ à $C_6$, les substituants dudit phényle substitué et de ladite moitié de phényle substituée du phénylalkyle de $C_7$ à $C_{10}$ sont choisis parmi un alkyle de $C_1$ à $C_4$ et un halo, et le substituant de ladite pipéridyle N-substitué est un alkyle de $C_1$ à $C_4$;

et des sels acceptables pharmaceutiquement de tels composés,

de telle façon, un composé de la formule générale II

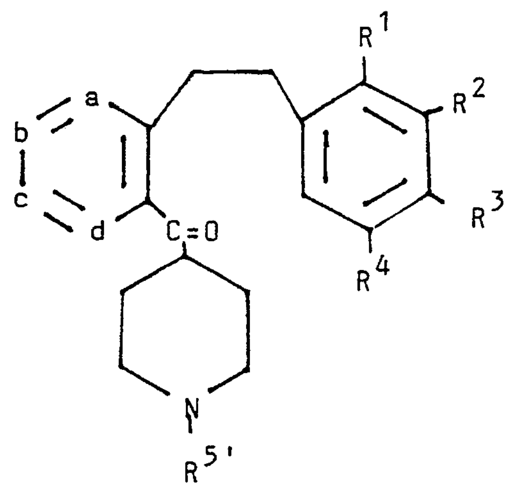

II

où a, b, c, d, $R^1$, $R^2$, $R^3$ et $R^4$ sont tels que définis ci-dessus, et

$R^{5'}$ est tel que défini pour $R^5$ ci-dessus ou représente un groupe protégeant M, est soumis à une condensation intramoléculaire suivie, si c'est nécessaire ou souhaité, par une ou plusieurs des étapes suivantes :

a) extraction d'un groupe quelconque de protection M représenté par $R^{5'}$;

b) transformation d'un composé où $R^5$ est un méthyle ou un -$COOR^7$ dans un composé où $R^5$ est un hydrogène;

c) transformation d'un composé où $R^5$ est un hydrogène dans un composé où $R^5$ est -$COOR^7$ ou un méthyle;

d) transformation d'un composé ou $R^5$ est un méthyle dans un composé où $R^5$ est $-COOR^7$; et

e) transformation d'un composé de formule I obtenu selon l'une quelconque des étapes ci-dessus dans un sel acceptable pharmaceutiquement;

caractérisé en ce que la condensation intramoléculaire est réalisée par le traitement du composé de formule II avec un super acide ayant une fonction d'acidité Hammett de moins de 12 ou plus basse.

2. Procédé selon la revendication 1, caractérisé en ce que l'acide a une fonction d'acidité Hammett de moins de 13 ou moins de 14, et en particulier est choisi parmi $HF/BF_3$, $CF_3SO_3H$, $CH_3SO_3H/BF_3$ et $FSO_3H$ .

3. Procédé de fabrication des composés de formule générale XIII

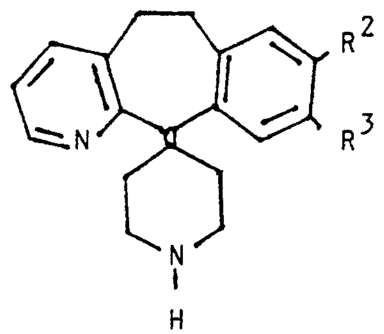

XIII

où $R^2$ est F, Br ou I et $R^3$ est H;

et leurs sels acceptables pharmaceutiquement, caractérisé en ce que

A : un composé de formule générale

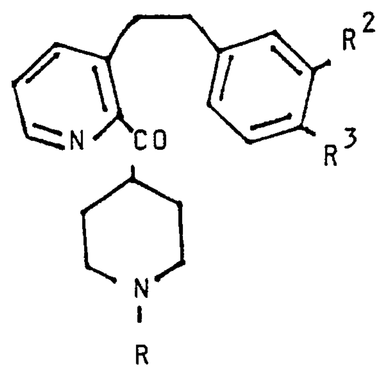

où $R^2$ et $R^3$ sont tels que définis ci-dessus et R est H ou un groupe protégeant N, est soumis à une condensation intramoléculaire; ou

B : un composé de formule générale

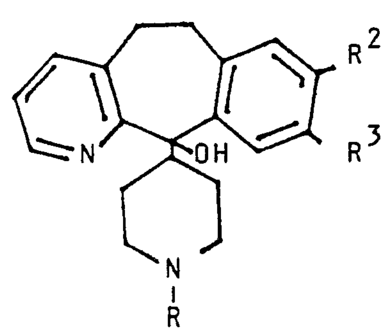

où $R^2$, $R^3$ et R sont tels que définis ci-dessus, est soumis à une déshydratation introduisant une double liaison entre la position 11 du noyau tricyclique et le résidu pipéridinyle;

suivi, si c'est nécessaire, par l'extraction d'un quelconque groupe protégeant M représenté par R et, si c'est souhaité, la transformation en un sel d'addition acide.

**4.** Procédé selon la revendication 3, où $R^2$ et $R^3$ peuvent être l'une des combinaisons suivantes :

| | $R^2$ | $R^3$ |
|---|---|---|
| 1. | H | I |
| 2. | $CF_3$ | H |
| 3. | F | F |
| 4. | Br | Br |
| 5. | I | I |
| 6. | $CF_3$ | $CF_3$ |
| 7. | F | Cl |

**5.** Procédé selon la revendication 3, où la condensation intramoléculaire est effectuée au moyen d'un acide, de préférence de l'acide sulfurique.

**6.** Procédé selon la revendication 4, où l'acide utilisé est un super acide tel que défini dans la revendication 1.

**7.** Procédé selon l'une quelconque des revendications 1 à 6, où le groupe protégeant M représenté par R est un alkyle de $C_1$ à $C_4$, de préférence un méthyle.

**8.** Procédé de préparation d'une composition pharmaceutique, caractérisé en ce qu'un composé de formule XIII tel que défini dans la revendication 3 est porté sous une forme appropriée pour une application pharmaceutique.

**9.** Utilisation d'un composé de formule XIII, tel que défini dans la revendication 3, pour la préparation de compositions pharmaceutiques ayant une activité anti-allergique.

**10.** Composition pharmaceutique comprenant, comme ingrédient actif, un composé de formule XIII défini dans la revendication 3 avec un porteur acceptable pharmaceutiquement.

## Ansprüche

**1.** Verfahren zur Herstellung von Verbindungen der allgemeinen Formel I

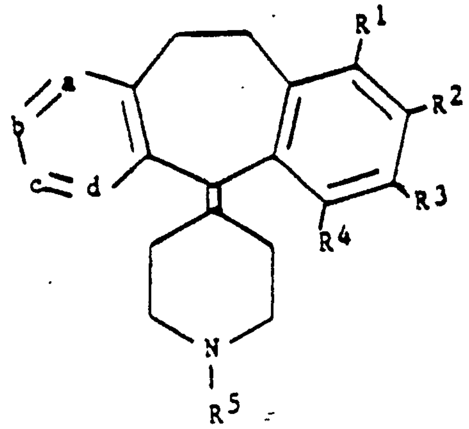

I

worin a, b, c und d CH bedeuten oder eines von a, b, c und d N bedeutet und die anderen CH bedeuten,

$R^1$, $R^2$, $R^3$ und $R^4$ gleich oder verschieden sein können und jeweils unabhängig voneinander Wasserstoff, Alkyl mit 1 bis 6 Kohlenstoff-Atomen, Fluor, Chlor, Brom, Iod, Nitro, Alkoxy mit 1 bis 6 Kohlenstoff-Atomen oder Trifluormethyl bedeuten;

$R^5$ Methyl, -H oder -$COOR^7$ ist, worin $R^7$ $C_1$ bis $C_{12}$ Alkyl, substituiertes $C_1$ bis $C_{12}$ Alkyl, $C_2$ bis $C_{12}$ Alkenyl, substituiertes $C_2$ bis $C_{12}$ Alkenyl, Phenyl, substituiertes Phenyl, $C_7$ bis $C_{10}$ Phenylalkyl oder $C_7$ bis $C_{10}$ Phenylalkyl, worin die Phenylhälfte substituiert ist, bedeutet, oder $R^7$ 2-, 3- oder 4-Piperidyl oder N-substituiertes Piperidyl ist, worin die Substituenten an dem substituierten $C_1$ bis $C_{12}$ Alkyl und an dem substituierten $C_2$ bis $C_{12}$ Alkenyl aus Amino und substituiertem Amino ausgewählt sind und die Substituenten an dem substituierten Amino aus $C_1$ bis $C_6$ Alkyl ausgewählt sind, die Substituenten an dem substituierten Phenyl und an der substituierten Phenylhälfte des $C_7$ bis $C_{10}$ Phenylalkyls aus $C_1$ bis $C_4$ Alkyl und Halo ausgewählt sind und die Substituenten an dem N-substituierten Piperidyl $C_1$ bis $C_4$ Alkyl sind;

und pharmazeutisch annehmbaren Salzen solcher Verbindungen,

wobei eine Verbindung der allgemeinen Formel II

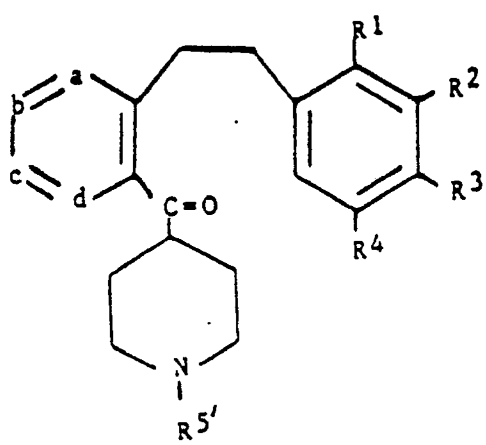

II

worin a, b, c, d, $R^1$, $R^2$, $R^3$ und $R^4$ die im Vorstehenden angegebenen Bedeutung haben, und $R^{5'}$ die im Vorstehenden für $R^5$ angegebene Bedeutung hat oder eine N-Schutzgruppe darstellt, einer intramolekularen Kondensation unterworfen wird, gefolgt, falls notwendig oder erwünscht, von einem oder mehreren der folgenden Schritte:

a) Entfernen jeglicher durch $R^{5'}$ dargestellter N-Schutzgruppen;

b) Überführung einer Verbindung, in der $R^5$ Methyl oder -$COOR^7$ ist, in eine Verbindung, in der $R^5$ Wasserstoff ist;

c) Überführung einer Verbindung, in der $R^5$ Wasserstoff ist, in eine Verbindung, in der $R^5$ -$COOR^7$ oder Methyl ist;

d) Überführung einer Verbindung, in der $R^5$ Methyl ist, in eine Verbindung, in der $R^5$ -$COOR^7$ ist;

und
e) Überführung einer gemäß einem der obigen Schritte erhaltenen Verbindung der Formel I in ein pharmazeutisch annehmbares Salz; dadurch gekennzeichnet, daß die intramolekulare Kondensation durch Behandeln der Verbindung der Formel II mit einer Supersäure mit einer Hammett-Säurefunktion von minus 12 oder weniger durchgeführt wird.

2. Verfahren gemäß Anspruch 1, dadurch gekennzeichnet, daß die Säure eine Hammett-Säurefunktion von minus 13 oder minus 14 hat.

3. Verfahren gemäß Anspruch 1 oder Anspruch 2, dadurch gekennzeichnet, daß die verwendete Säure aus $HF/BF_3$, $CF_3SO_3H$ , $CH_3SO_3H/BF_3$ und $FSO_3H$ ausgewählt ist.

4. Verbindungen der allgemeinen Formel XIII

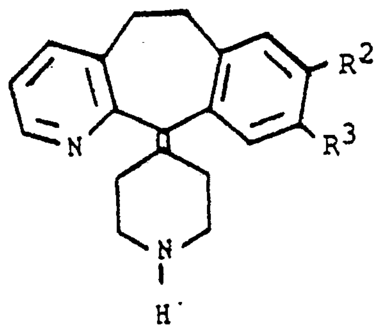

XIII

worin $R^2$ F, Br oder I ist und $R^3$ H ist, und deren pharmazeutisch annehmbare Salze und weiter umfassend die Verbindungen, in denen $R^2$ und $R^3$ eine der folgenden Kombinationen sein können:

| | $R^2$ | $R^3$ |
|---|---|---|
| 1. | H | I |
| 2. | $CF_3$ | H |
| 3. | F | F |
| 4. | Br | Br |
| 5. | I | I |
| 6. | $CF_3$ | $CF_3$ |
| 7. | F | Cl. |

5. Verbindungen wie in Anspruch 4 beansprucht, in denen $R^2$ F ist und $R^3$ H ist.

6. Pharmazeutische Zusammensetzung unfassend als aktiven Bestandteil eine in Anspruch 4 definierte Verbindung der Formel XIII zusammen mit einem pharmazeutisch annehmbaren Träger.

7. Verwendung einer Verbindung wie in Anspruch 4 oder Anspruch 5 definiert zur Herstellung von pharmazeutischen Zusammensetzungen mit antiallergischer Wirksamkeit.

Patentansprüche für folgenden Vertragsstaat:AT

1. Verfahren zur Herstellung von Verbindungen der allgemeinen Formel I

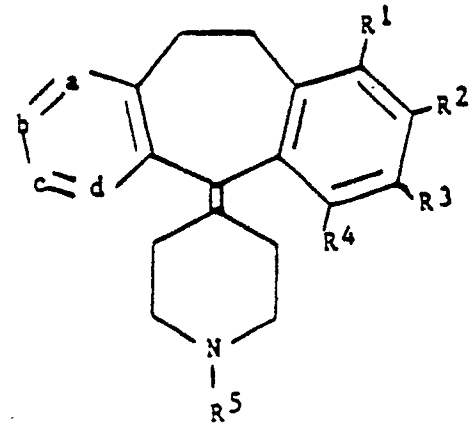

I

worin a, b, c und d CH bedeuten oder eines von a, b, c und d N bedeutet und die anderen CH bedeuten,

$R^1$, $R^2$, $R^3$ und $R^4$ gleich oder verschieden sein können und jeweils unabhängig voneinander Wasserstoff, Alkyl mit 1 bis 6 Kohlenstoff-Atomen, Fluor, Chlor, Brom, Iod, Nitro, Alkoxy mit 1 bis 6 Kohlenstoff-Atomen oder Trifluormethyl bedeuten;

$R^5$ Methyl, -H oder -COOR$^7$ ist, worin $R^7$ $C_1$ bis $C_{12}$ Alkyl, substituiertes $C_1$ bis $C_{12}$ Alkyl, $C_2$ bis $C_{12}$ Alkenyl, substituiertes $C_2$ bis $C_{12}$ Alkenyl, Phenyl, substituiertes Phenyl, $C_7$ bis $C_{10}$ Phenylalkyl oder $C_7$ bis $C_{10}$ Phenylalkyl, worin die Phenylhälfte substituiert ist, bedeutet, oder $R^7$ 2-, 3- oder 4-Piperidyl oder N-substituiertes Piperidyl ist, worin die Substituenten an dem substituierten $C_1$ bis $C_{12}$ Alkyl und an dem substituierten $C_2$ bis $C_{12}$ Alkenyl aus Amino und substituiertem Amino ausgewählt sind und die Substituenten an dem substituierten Amino aus $C_1$ bis $C_6$ Alkyl ausgewählt sind, die Substituenten an dem substituierten Phenyl und an der substituierten Phenylhälfte des $C_7$ bis $C_{10}$ Phenylalkyls aus $C_1$ bis $C_4$ Alkyl und Halo ausgewählt sind und die Substituenten an dem N-substituierten Piperidyl $C_1$ bis $C_4$ Alkyl sind;

und pharmazeutisch annehmbaren Salzen solcher Verbindungen,

wobei eine Verbindung der allgemeinen Formel II

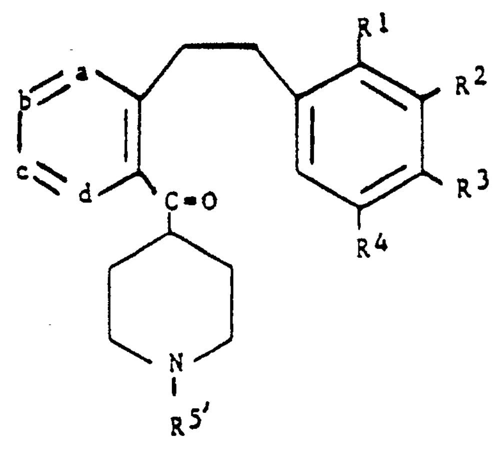

II

worin a, b, c, d, $R^1$, $R^2$, $R^3$ und $R^4$ die im Vorstehenden angegebene Bedeutung haben, und $R^{5'}$ die im Vorstehenden für $R^5$ angegebene Bedeutung hat oder eine N-Schutzgruppe darstellt, einer intramolekularen Kondensation unterworfen wird, gefolgt, falls notwendig oder erwünscht, von einem oder mehreren der folgenden Schritte:

a) Entfernen jeglicher durch $R^{5'}$ dargestellter N-Schutzgruppen;

b) Überführung einer Verbindung, in der $R^5$ Methyl oder -COOR$^7$ ist, in eine Verbindung, in der $R^5$ Wasserstoff ist;

c) Überführung einer Verbindung, in der $R^5$ Wasserstoff ist, in eine Verbindung, in der $R^5$ -COOR$^7$ oder Methyl ist;

d) Überführung einer Verbindung, in der $R^5$ Methyl ist, in eine Verbindung, in der $R^5$ $-COOR^7$ ist, und

e) Überführung einer gemäß einem der obigen Schritte erhaltenen Verbindung der Formel I in ein pharmazeutisch annehmbares Salz; dadurch gekennzeichnet, daß die intramolekulare Kondensation durch Behandeln der Verbindung der Formel II mit einer Supersäure mit einer Hammett-Säurefunktion von minus 12 oder weniger durchgeführt wird.

**2.** Verfahren gemäß Anspruch 1, dadurch gekennzeichnet, daß die Säure eine Hammett-Säurefunktion von minus 13 oder minus 14 hat und insbesondere aus $HF/BF_3$, $CF_3SO_3H$, $CH_3SO_3H/BF_3$ und $FSO_3H$ ausgewählt ist.

**3.** Verfahren zur Herstellung von Verbindungen der allgemeinen Formel XIII

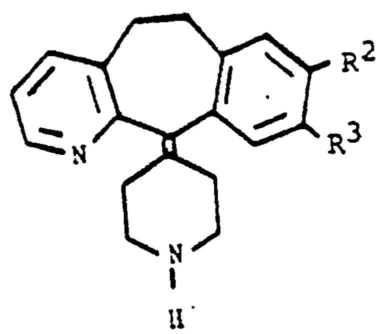

XIII

worin $R^2$ F, Br oder I ist und $R^3$ H ist, und deren pharmazeutisch annehmbaren Salzen, dadurch charakterisiert, daß

A: eine Verbindung der allgemeinen Formel

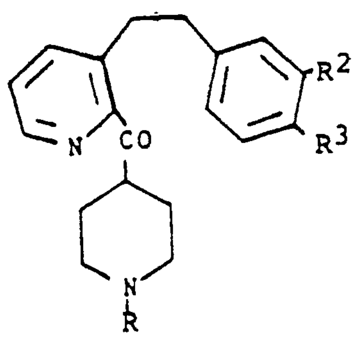

worin $R^2$ und $R^3$ die im Vorstehenden angegebene Bedeutung haben und R H oder eine N-Schutzgruppe ist, einer intramolekularen Kondensation unterworfen wird, oder B: eine Verbindung der allgemeinen Formel

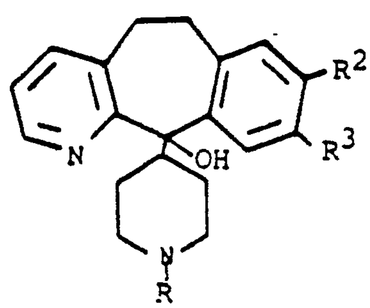

worin $R^2$, $R^3$ und R die im Vorstehenden angegebene Bedeutung haben, einer Dehydrierung unterworfen wird, welche eine Doppelbindung zwischen Position 11 des tricyclischen Kerns und dem Piperidinylrest einführt,

gefolgt, falls nötig, vom Entfernen jeglicher durch R dargestellter N-Schutzgruppen und, falls gewünscht, der Ümwandlung in ein Säureadditionssalz.

4. Verfahren gemäß Anspruch 3, wobei $R^2$ und $R^3$ eine der folgenden Kombinationen sein können:

| | $R^2$ | $R^3$ |
|---|---|---|
| 1. | H | I |
| 2. | $CF_3$ | H |
| 3. | F | F |
| 4. | Br | Br |
| 5. | I | I |
| 6. | $CF_3$ | $CF_3$ |
| 7. | F | Cl. |

5. Verfahren gemäß Anspruch 3, wobei die intramolekulare Kondensation mittels einer Säure, vorzugsweise Schwefelsäure durchgeführt wird.

6. Verfahren gemäß Anspruch 4, wobei die verwendete Säure eine wie in Anspruch 1 definierte Supersäure ist.

7. Verfahren nach irgendeinem der Ansprüche 1 bis 6, wobei die durch R dargestellte N-Schutzgruppe $C_1$-$C_4$-Alkyl , vorzugsweise Methyl, ist.

8. Verfahren zur Herstellung einer pharmazeutischen Zusammensetzung, dadurch gekennzeichnet, daß eine wie in Anspruch 3 definierte Verbindung der Formel XIII in eine für die pharmazeutische Anwendung geeignete Form gebracht wird.

9. Verwendung einer wie in Anspruch 3 definierten Verbindung der Formel XIII zur Herstellung pharmazeutischer Zusammensetzungen mit antiallergischer Wirksamkeit.

10. Pharmazeutische Zusammensetzung umfassend als aktiven Bestandteil eine wie in Anspruch 3 definierte Verbindung der Formel XIII zusammen mit einem pharmazeutisch annehmbaren Träger.